(19) Europäisches Patentamt
European Patent Office
Office européen des brevets

(11) Veröffentlichungsnummer: **0 019 811**
**A1**

(12) # EUROPÄISCHE PATENTANMELDUNG

(21) Anmeldenummer: **80102724.4**

(22) Anmeldetag: **16.05.80**

(51) Int. Cl.³: **C 07 D 403/12**, C 07 D 239/42, A 61 K 31/505, C 07 D 239/48, C 07 D 239/46, C 07 D 239/52, C 07 D 239/54

(30) Priorität: **18.05.79 CH 4669/79**

(43) Veröffentlichungstag der Anmeldung: **10.12.80 Patentblatt 80/25**

(84) Benannte Vertragsstaaten: **AT BE CH DE FR IT LI LU NL SE**

(71) Anmelder: **CIBA-GEIGY AG, Patentabteilung Postfach, CH-4002 Basel (CH)**

(72) Erfinder: **Marxer, Adrian, Prof. Dr., Rieserstrasse 4, CH-4132 Muttenz (CH)**
Erfinder: **Eichenberger, Kurt, Dr., Neubergweg 36, CH-4106 Therwil (CH)**

(74) Vertreter: **Zumstein sen., Fritz, Dr. et al, Bräuhausstrasse 4, D-8000 München 2 (DE)**

(54) **Pyrimidin-Verbindungen, Verfahren zu ihrer Herstellung, pharmazeutische Präparate enthaltend diese Verbindungen, sowie ihre therapeutische Verwendung.**

(57) Die Erfindung betrifft antihypertensive Verbindungen der Formel I

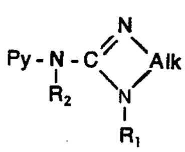

(I),

worin Py einen gegebenenfalls substituierten, über ein Kohlenstoffatom mit dem Stickstoffatom verbundenen 4- oder 5-Pyrimidinylrest darstellt, $R_1$ und $R_2$ unabhängig voneinander Wasserstoff, Niederalkyl oder Niederalkenyl bedeuten, und Alk für Niederalkylen steht, das die beiden Stickstoffatome durch 2 bis 4 Kohlenstoffatome trennt, ihre tautomeren Verbindungen und Säureadditionssalze. Sie können z.B. durch Umsetzung einer Verbindung der Formel Py-N($R_2$)H mit einem Salz einer Verbindung der Formel

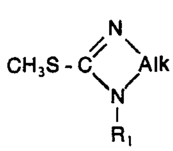

hergestellt werden.

EP 0 019 811 A1

CIBA-GEIGY AG

Basel (Schweiz)

4-12359/+

Pyrimidin-Verbindungen, Verfahren zu ihrer Herstellung, pharmazeutische Präparate enthaltend diese Verbindungen, sowie ihre therapeutische Verwendung.

Die vorliegende Erfindung betrifft 2-(Pyrimidinylamino)-1,3-diaza-2-cycloalken-Verbindungen, insbesondere der Formel

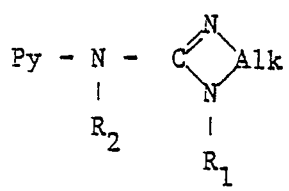

(I),

worin Py einen gegebenenfalls substituierten, über ein Kohlenstoffatom mit dem Stickstoffatom verbundenen 4- oder 5-Pyrimidinylrest darstellt, $R_1$ und $R_2$ unabhängig voneinander Wasserstoff, Niederalkyl oder Niederalkenyl bedeuten, und Alk für Niederalkylen steht, das die beiden Stickstoffatome durch 2 bis 4 Kohlenstoffatome trennt, ihre tautomeren Verbindungen und Salze, sowie Verfahren zu ihrer Herstellung, die Verbindungen der Formel I und ihre Salze als pharmakologisch aktive Verbindungen, pharmazeutische Präparate enthaltend solche Verbindungen, und die Verwendung der neuen Verbindungen als pharmakologisch wirksame Substanzen, sowie zur Herstellung von pharmazeutischen Präparaten.

Der Pyrimidinylrest Py stellt einen 4- oder 5-, vorzugsweise einen 4-Pyrimidinylrest dar, der z.B. Niederalkyl, Cycloalkyl, Hydroxy,

Niederalkoxy, Niederalkylthio, Halogen, Trifluormethyl, Niederalkylsulfonyl, gegebenenfalls substituiertes Phenyl oder Phenoxy, und/oder gegebenenfalls substituiertes Amino als Substituenten enthalten kann.

Im Zusammenhang mit der vorliegenden Beschreibung mit "nieder" bezeichnete Reste und Verbindungen enthalten vorzugsweise bis zu 7, in erster Linie bis zu 4 Kohlenstoffatome.

Niederalkyl steht, z.B. insbesondere für Methyl, sowie für Aethyl, n-Propyl, Isopropyl, n-Butyl, Isobutyl oder tert.-Butyl, sowie n-Pentyl, Neopentyl, n-Hexyl oder n-Heptyl.

Niederalkenyl steht z.B. insbesondere für Allyl, eine 1-, 2- oder 3-Methylallylgruppe.

Niederalkoxy ist z.B. in erster Linie Methoxy, kann aber auch Aethoxy, n-Propyloxy, Isopropyloxy oder n-Butyloxy, ferner n-Pentyloxy sein.

Niederalkylthio ist insbesondere Methylthio, ferner auch Aethylthio, Isopropylthio, n-Propylthio oder auch ein gerades oder verzweigtes Butylthio.

Nideralkylsulfonyl ist z.B. Methylsulfonyl, Aethylsulfonyl, n-Propylsulfonyl oder Isopropylsulfonyl.

Ein Cycloalkylrest ist in erster Linie eine Cycloalkylgruppe mit 3-6 Ringkohlenstoffatomen und ist z.B. eine Cyclopropyl-, Cyclobutyl-, Cyclopentyl- oder Cyclohexylgruppe.

Halogen ist insbesondere Halogen mit Atomnummer bis zu 35 und steht in erster Linie für Chlor, ferner für Fluor oder Brom.

Ein gegebenenfalls substituierter Phenyl- oder Phenoxyrest kann durch einen, zwei oder drei gleiche oder verschiedene Substituenten

substituiert sein. Solche Substituenten sind z.B. Niederalkyl, gegebenenfalls funktionell abgewandeltes Hydroxy oder Mercapto, wie veräthertes Hydroxy, z.B. Niederalkoxy, Niederalkenyloxy oder Niederalkylendioxy, ferner Niederalkylthio, oder Halogen, Trifluormethyl, Nitro, Amino einschliesslich substituiertes Amino, z.B. Niederalkylamino oder Diniederalkylamino, gegebenenfalls funktionell abgewandeltes Carboxy, wie verestertes Carboxy, z.B.Niederalkoxycarbonyl.

Niederalkenyloxy ist z.B. in erster Linie Vinyloxy oder Allyloxy.

Gegebenenfalls substituiertes Amino kann durch Niederalkyl oder gegebenenfalls substituiertes Phenyl substituiert sein und ist z.B. Niederalkylamino oder Diniederalkylamino, wie Methylamino, Aethylamino, Dimethylamino oder Diäthylamino, Phenylamino, 4-Methoxy-phenylamino oder 4-Chlor-phenylamino. Es kann auch durch, gegebenenfalls Sauerstoff, Schwefel oder gegebenenfalls Niederalkyl enthaltendes Stickstoff als Ringglied aufweisendes Niederalkylen substituiert sein und z.B. Niederalkylenamino, z.B. Pyrrolidino oder Piperidino, Oxaniederalkylenamino, z.B. Morpholino, Thianiederalkylenamino, z.B. Thiomorpholino oder Azaniederalkylenamino, z.B. Piperazino oder 4-Niederalkyl-piperazino, wie 4-Methyl-piperazino, darstellen. Substituiertes Amino kann auch Acylamino, wie Niederalkanoylamino, z.B. Acetylamino oder Propionylamino, Niederalkoxycarbonylamino, z.B. Methoxycarbonylamino oder Aethoxycarbonylamino, oder gegebenenfalls durch Niederalkyl substituiertes Ureido, z.B. Ureido, 3-Methylureido oder 3,3-Dimethylureido, sein.

Eine Niederalkylengruppe Alk ist vorzugsweise unverzweigtes Niederalkylen und steht in erster Linie für Aethylen, sowie für 1,3-Propylen oder 1,4-Butylen, kann aber auch verzweigtes Niederalkylen, wie 1,2-Propylen, 2-Methyl-1,2-propylen oder 2,3-Butylen darstellen.

Salze von Verbindungen der obigen Formel I sind Säureadditionssalze, insbesondere pharmazeutisch verwendbare, nicht-toxische Säureadditionssalze mit anorganischen Säuren, z.B. Chlorwasserstoffsäure, Bromwasserstoffsäure, Schwefelsäure oder Phosphorsäuren, oder mit organischen Säuren, wie entsprechenden Carbonsäuren, z.B. Essigsäure, Propionsäure, Glykolsäure, Bernsteinsäure, Maleinsäure, Hydroxymaleinsäure, Methylmaleinsäure, Fumarsäure, Aepfelsäure, Weinsäure, Zitronensäure, Benzoesäure, Zimtsäure, Mandelsäure, Salicylsäure, 4-Aminosalicylsäure, 2-Phenoxybenzoesäure, 2-Acetoxy-benzoesäure, Embonsäure, Nicotinsäure oder Isonicotinsäure, oder Sulfonsäuren, z.B. Methansulfonsäure, Aethansulfonsäure, 2-Hydroxyäthansulfonsäure, Aethan-1,2-disulfonsäure, Benzolsulfonsäure, 4-Methyl-benzol-sulfonsäure oder Naphthalin-2-sulfonsäure.

Infolge der engen Beziehung zwischen den neuen Verbindungen in freier Form und in Form ihrer Salze, inkl. auch solcher Säureadditionssalze, die als Zwischenprodukte, z.B. bei der Reinigung der neuen Verbindungen oder zu ihrer Identifikation verwendet werden können, sind vorausgehend und nachfolgend unter den freien Verbindungen sinn- und zweckmässig gegebenenfalls auch die entsprechenden Salze zu verstehen.

Die Verbindungen der vorliegenden Erfindung weisen wertvolle Eigenschaften, in erster Linie pharmakologische Wirkungen auf. So zeigen sie hypotensive und antihypertensive Wirkungen, die sich an anästhesierten Katzen in Dosen ab etwa 0,1 mg/kg bei intravenöser Verabreichung (wobei auch die pressorischen Wirkungen von Adrenalin und Noradrenalin antagonisiert werden) und an renal hypertonischen Ratten in Dosen ab etwa 10 mg/kg/Tag bei oraler Verabreichung nachweisen lassen. Ferner wirken die erfindungsgemässen Verbindungen auf die Herztätigkeit, was anhand der am isolierten Meerschweinchenvorhof bei Konzentrationen von 100 µg/ml festgestellten positiv inotropen und in Konzentrationen ab 10 µg/ml festgestellten negativ chronotropen Wirkungen nachgewiesen werden kann. Dabei weisen die Verbindungen der Formel I einen günstigen therapeutischen Index, d.h. ein günstiges

Verhältnis zwischen effektiver und toxischer Dosis, auf. Die Verbindungen der vorliegenden Erfindung werden deshalb als pharmakologisch wirksame Verbindungen, in erster Linie als Antihypertensiva zur Behandlung von erhöhtem Blutdruck, z.B. im Zusammenhang mit essentieller Hypertonie, und als Kardiotonika verwendet.

Die Erfindung betrifft in erster Linie Verbindungen der Formel I, worin Py für gegebenenfalls durch einen, zwei oder drei gleiche oder verschiedene Substituenten der Gruppe Niederalkyl, Hydroxy, Niederalkoxy, Niederalkylthio, Halogen, Trifluormethyl, Niederalkylsulfonyl, Amino, gegebenenfalls durch Niederalkyl, Niederalkoxy, Hydroxy, Amino, Niederalkylamino, Diniederalkylamino oder Halogen substituiertes Phenyl, Phenoxy oder Phenylamino, Niederalkylamino, Diniederalkylamino, Pyrrolidino, Piperidino, Morpholino, Thiomorpholino, Niederalkanoylamino, Niederalkoxycarbonylamino, Ureido, 3-Niederalkylureido und 3,3-Diniederalkylureido substituiertes, über ein Kohlenstoffatom mit dem Stickstoffatom verbundenes 4- oder 5-Pyrimidinyl, insbesondere 4-Pyrimidinyl steht, $R_1$ und $R_2$ unabhängig voneinander Wasserstoff, Niederalkyl oder Niederalkenyl bedeuten, und Alk für Niederalkylen steht, das die beiden Stickstoffatome durch 2 bis 4 Kohlenstoffatome trennt, wobei mit "nieder" bezeichnete Reste bis zu 4 Kohlenstoffatomen enthalten, ihre tautomeren Verbindungen und Salze, insbesondere pharmazeutisch verwendbare, nicht-toxische Säureadditionssalze davon.

Die Erfindung betrifft in erster Linie Verbindungen der Formel I, worin Py für gegebenenfalls durch einen, zwei oder drei gleiche oder verschiedene Substituenten der Gruppe Niederalkyl, Niederalkoxy, Phenyl, Amino, Niederalkylamino, Diniederalkylamino oder Morpholino und/oder Halogen substituiertes, über ein Kohlenstoffatom mit dem Stickstoffatom verbundenes 4- oder 5-Pyrimidinyl, insbesondere 4-Pyrimidinyl steht, $R_1$ Wasserstoff oder Niederalkyl und $R_2$ Wasserstoff oder Niederalkyl darstellen, und Alk für Niederalkylen steht, das die beiden Stickstoffatome durch 2 bis 3 Kohlenstoffatome trennt, wobei mit "nieder" bezeichnete Reste bis zu 4 Kohlenstoffatomen enthalten, und Halogen ein

Atomgewicht bis zu 35 aufweist, und Salze, insbesondere pharmazeutisch verwendbare, nicht-toxische Säureadditionssalze davon.

Die Erfindung betrifft insbesondere Verbindungen der Formel

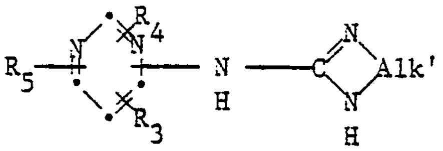

(II),

worin Alk' für Niederalkylen mit bis zu 4 Kohlenstoffatomen, das die beiden Stickstoffatome durch 2 bis 3 Kohlenstoffatome trennt, insbesondere für Aethylen steht, und jeder der Reste $R_3$, $R_4$ und $R_5$ Wasserstoff, Niederalkyl mit bis zu 4 Kohlenstoffatomen, z.B. Methyl, oder Niederalkoxy mit bis zu 4 Kohlenstoffatomen, z.B. Methoxy, oder Halogen, wie z.B. Chlor oder Brom, oder Diniederalkylamino, wie z.B. Dimethylamino oder Diäthylamino, Morpholino oder Phenyl, bedeutet, wobei vorzugsweise mindestens einer der Reste $R_3$, $R_4$ und $R_5$, vorzugsweise jedoch zwei davon, von Wasserstoff verschieden ist, bedeuten, und Salze, insbesondere pharmazeutisch verwendbare, nicht-toxische Säureadditionssalze davon.

Die Erfindung betrifft insbesondere Verbindungen der Formel

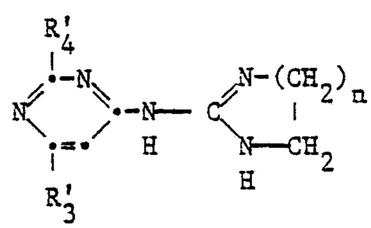

(III),

worin $R_3'$ und $R_4'$ unabhängig voneinander für Wasserstoff, Niederalkyl mit bis zu 4 Kohlenstoffatomen, z.B. Methyl, oder Niederalkoxy mit bis zu 4 Kohlenstoffatomen, z.B. Methoxy, Halogen, z.B. Chlor, Diniederalkylamino, z.B. Dimethylamino stehen, wobei vorzugsweise beide der Reste $R_3'$ und $R_4'$ von Wasserstoff verschieden sind und in erster Linie Niederalkyl, z.B. Methyl, Niederalkoxy, z.B. Methoxy, Halogen, z.B. Chlor oder Diniederalkylamino, z.B. Dimethylamino bedeuten, und n für 2 und in erster Linie für 1 steht, und Salze, insbesondere pharmazeutisch verwendbare, nicht-toxische Säureadditionssalze davon.

Die Erfindung betrifft in erster Linie die in den Beispielen beschriebenen Verbindungen der Formel I und Salze, insbesondere pharmazeutisch verwendbare, nicht-toxische Säureadditionssalze davon.

Die Verbindungen der vorliegenden Erfindung können nach an sich bekannten Methoden hergestellt werden, z.B. indem man eine Verbindung der Formel Py-X (IV) oder ein Salz davon mit einer Verbindung der Formel

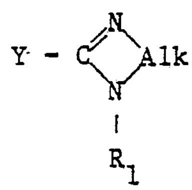

(V)

oder einem Salz davon umsetzt, worin einer der Reste X und Y eine Aminogruppe der Formel $-N(R_2)-H$ (VI) darstellt, und der andere eine unter den Reaktionsbedingungen zusammen mit Wasserstoff abspaltbare Gruppe bedeutet, und, wenn erwünscht, eine erhaltene Verbindung der Formel I in eine andere Verbindung der Formel I überführt, und/oder, wenn erwünscht, ein erhaltenes Salz in die freie Verbindung oder in ein anderes Salz überführt, und/oder, wenn erwünscht, eine erhaltene freie Verbindung in ein Salz überführt, und/oder, wenn erwünscht, ein erhaltenes Gemisch von Isomeren in die einzelnen Isomeren auftrennt.

Eine zusammen mit Wasserstoff abspaltbare Gruppe X bzw. Y ist z.B. in erster Linie eine freie oder vorzugsweise verätherte Mercaptogruppe, ferner eine reaktionsfähige, funktionell abgewandelte Hydroxygruppe oder die Nitroaminogruppe. Eine verätherte Mercaptogruppe ist in erster Linie eine durch einen gegebenenfalls substituierten Kohlenwasserstoffrest, insbesondere aliphatischen Charakters, verätherte Mercaptogruppe. Sie stellt in erster Linie Niederalkylthio, z.B. Methylthio, Aethylthio oder Butylthio oder Phenylniederalkylthio, z.B. Benzylthio, dar. Eine reaktionsfähige, funktionell abgewandelte Hydroxygruppe ist eine entsprechende verätherte oder veresterte

Hydroxygruppe. Eine solche ist u.a. Niederalkoxy, z.B. Methoxy, oder Halogen, z.B. Chlor oder Brom, oder Niederalkylsulfonyloxy, z.B. Methansulfonyloxy.

Vorzugsweise bedeutet in einer Verbindung der Formel IV die Gruppe X die Aminogruppe der Formel VI, während in einer Verbindung der Formel V der Rest Y in erster Linie für eine verätherte Mercaptogruppe, insbesondere Niederalkylthio, z.B. Methylthio, steht.

Salze von Ausgangsstoffen der Formeln IV bzw. V sind Säureadditionssalze, z.B. Salze mit den obgenannten Säuren, insbesondere mit Mineralsäuren, wie Halogenwasserstoffsäuren, z.B. Salzsäure, Jodwasserstoffsäure oder Schwefelsäure. Dabei wird insbesondere das von einer Aminoverbindung der Formel IV bzw. V verschiedene Ausgangsmaterial und in erster Linie ein Ausgangsmaterial der Formel V, worin Y für eine gegebenenfalls verätherte Mercaptogruppe oder für eine reaktionsfähige, funktionell abgewandelte Hydroxygruppe steht, in der Form eines Säureadditionssalzes verwendet.

Die obige Reaktion wird in an sich bekannter Weise durchgeführt, z.B. in Ab- oder Anwesenheit eines Lösungsmittels oder eines Lösungsmittelgemisches, falls erwünscht, in Gegenwart eines Ueberschusses der als Ausgangsmaterial eingesetzten Aminkomponente, unter Kühlen oder vorzugsweise unter Erwärmen, z.B. bei einer Temperatur von etwa 50°C bis etwa 180°C, vorzugsweise bei 160°C, wenn notwendig, in einem geschlossenen Gefäss, gegebenenfalls unter Druck, und/oder unter einer Inertgas-, z.B. Stickstoffatmosphäre.

Die Ausgangsstoffe sind bekannt oder können in an sich bekannter Weise hergestellt werden.

Die neuen Verbindungen können ebenfalls hergestellt werden, indem man eine Verbindung der Formel

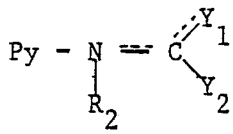

(VII)

worin $Y_1$ die Iminogruppe, eine abspaltbare Gruppe, die Oxo- oder Thioxogruppe bedeutet, und $Y_2$ eine abspaltbare Gruppe oder $Y_1$ und $Y_2$ zusammengenommen ein dreifach gebundenes Stickstoffatom bedeuten, wenn $R_2$ die Bedeutung von Wasserstoff hat, oder die entsprechende tautomere Form, oder ein Salz davon, mit einer Alkylendiamin-Verbindung der Formel $H_2N\text{-}Alk\text{-}NHR_1$ (VIII) umsetzt, und, wenn erwünscht, zusätzliche Verfahrensschritte durchführt.

Eine abspaltbare Gruppe ist bereits vorher unter Formel IV oder V in Zusammenhang mit den Substituenten X oder Y definiert worden. Eine Verbindung der Formel VII wird üblicherweise in der Form eines Säureadditionssalzes, insbesondere eines Salzes mit einer Mineralsäure, wie einer Halogenwasserstoffsäure, z.B. Chlor-, Brom- oder Jodwasserstoffsäure, eingesetzt. Die Kondensation zum Ring kann in einer oder zwei Stufen erfolgen.

Die obige Reaktion kann in Ab- oder Anwesenheit eines Lösungsmittels, wie eines vorzugsweise polaren Lösungsmittels, durchgeführt werden. Dabei arbeitet man bei Raumtemperatur oder vorzugsweise bei erhöhten Temperaturen, z.B. bei etwa 50°C bis etwa 200°C, wobei bei Abwesenheit eines Lösungsmittels das Gemisch der beiden Reaktionsteilnehmer (die Verbindung der Formel VII vorzugsweise in Form eines Säureadditionssalzes, und eine Verbindung der Formel VIII vorzugsweise im Ueberschuss) auf Temperaturen von etwa 100°C bis etwa 200°C erhitzt wird. Die Reaktion kann in einem geschlossen Gefäss, gegebenenfalls unter erhöhtem Druck, und/oder unter einer Inertgas-, wie Stickstoffatmosphäre, vorgenommen werden.

Die Ausgangsstoffe sind bekannt oder können in an sich bekannter Weise, beispielsweise diejenigen der Formel VII, z.B. durch Behandeln einer Aminverbindung der Formel Py-NH-$R_2$ (IVa) mit einer geeigneten Isocyanat- oder Isothiocyanat-Verbindung, wie einem Acyl-isocyanat oder -isothiocyanat, z.B. einem Niederalkoxycarbonyl-, wie Aethoxycarbonylisocyanat oder -isothiocyanat, oder einem Aroyl-, wie Benzoylisocyanat oder -isothiocyanat (wobei diese gegebenenfalls in situ, z.B. durch Behandeln eines Alkalimetall- oder Ammoniumcyanats oder -thiocyanats mit einem entsprechenden Säurehalogenid, z.B. -chlorid, oder einem geeigneten Säureester, hergestellt werden), Entfernen der Acylgruppe aus einer erhaltenen N-Acyl-harnstoff- oder N-Acyl-thioharnstoff-Verbindung durch Hydrolyse, vorzugsweise in Gegenwart eines alkalischen Mittels, z.B. Natriumhydroxid, und Umwandeln des entsprechenden Harnstoff- bzw. Thioharnstoff-Zwischenprodukts in die gewünschte O- bzw. S-substituierte Isoharnstoff- bzw. Isothioharnstoff-Verbindung der Formel VII durch Behandeln mit einem reaktionsfähigen Ester eines Alkohols, wie mit einem Niederalkylhalogenid, z.B. Methylchlorid, -bromid oder -iodid, oder einem Diniederalkylsulfat, z.B. Dimethylsulfat, hergestellt werden.

In einer besonderen Verfahrensvariante des vorherigen Verfahrens kann man die neuen Verbindungen der Formel I ebenfalls erhalten, wenn man eine Verbindung der Formel

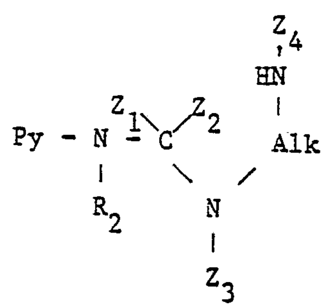

(IX),

worin $Z_1$ und $Z_2$ zusammen für Sauerstoff oder Schwefel stehen, und $Z_3$ den Rest $R_1$ und $Z_4$ Wasserstoff bedeuten, oder $Z_1$ für eine abspaltbare Gruppe steht, $Z_2$ und $Z_3$ zusammen eine Bindung bilden, und $Z_4$ den Rest $R_1$ bedeutet, ringschliesst, und, wenn erwünscht, zusätzliche Verfahrensschritte durchführt.

Der Ringschluss des obigen Ausgangsmaterials der Formel IX kann mittels Pyrolyse durchgeführt werden, wobei man bei Temperaturen von etwa 100°C bis etwa 200°C, wenn notwendig oder erwünscht, in Anwesenheit eines geeigneten hochsiedenden Lösungsmittels, in einem geschlossenen Gefäss, gegebenenfalls unter erhöhtem Druck, und/oder unter einer Inertgas-, z.B. Stickstoffatmosphäre, arbeitet.

Das Ausgangsmaterial kann in an sich bekannter Weise hergestellt werden, z.B. durch Behandeln einer Verbindung der Formel Py-N=C=Z (X) oder der Formel Py-N($R_2$)-C(=Z)-$Hal_1$ (XI), worin $Hal_1$ für Halogen, insbesondere Chlor und Z für Sauerstoff oder Schwefel steht, mit einer Alkylendiamin-Verbindung der Formel $H_2N$-Alk-NH-$R_1$ (VIII).

Die neuen Verbindungen der Formel I können ebenfalls erhalten werden, indem man ein Phosphinsäurehalogenid der Formel

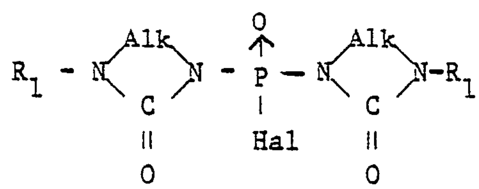

(XII) ,

worin Hal Halogen bedeutet, mit einer Amin-Verbindung der Formel Py-NH-$R_2$ (IVa) umsetzt, und, wenn erwünscht, zusätzliche Verfahrensschritte durchführt.

In einem Ausgangsmaterial der Formel XII ist Hal in erster Linie Chlor, und die Reaktion mit der Aminverbindung der Formel IVa wird in Abwesenheit, üblicherweise jedoch in Anwesenheit eines Lösungsmittels, wie eines hochsiedenden Lösungsmittels, z.B. Xylol, vorzugsweise bei erhöhter Temperatur, z.B. bei etwa 100°C bis etwa 200°C, wenn notwendig, in einem geschlossenen Gefäss, gegebenenfalls unter erhöhtem Druck, oder in einer Inertgas-, z.B. Stickstoffatmosphäre, durchgeführt.

Die Ausgangsstoffe sind bekannt und können in an sich bekannter Weise hergestellt werden. So erhält man z.B. Verbindungen der Formel XII, indem man eine Verbindung der Formel

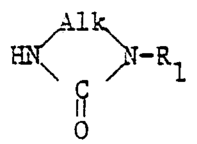

(XIII)

mit Phosphoroxychlorid, z.B. in Gegenwart von Chloroform als Lösungsmittel, bei einer Temperatur von etwa 20°C bis etwa 40°C umsetzt.

Wenn erwünscht, können Verbindungen der Formel I in andere Verbindungen der Formel I übergeführt werden. So kann man z.B. in Verbindungen der Formel I, worin $R_1$ und/oder $R_2$ Wasserstoff bedeuten, diesen in an sich bekannter Weise, z.B. durch Behandeln der entsprechenden Verbindung mit einem reaktionsfähigen Ester eines Niederalkanols, wie mit einem Niederalkylhalogenid, z.B. Methyl- oder Aethylchlorid, -bromid oder -iodid, oder einem Di-niederalkyl-sulfat, z.B. Dimethylsulfat, durch Niederalkyl, z.B. Methyl oder Aethyl, ersetzen. Dabei arbeitet man in Ab- oder vorzugsweise in Anwesenheit eines Lösungsmittels, falls notwendig, unter Kühlen oder Erwärmen, z.B. in einem Temperaturbereich von etwa 0°C bis etwa 100°C, in einem geschlossenen Gefäss, gegebenenfalls unter erhöhtem Druck, und/oder in einer Inertgas-, z.B. Stickstoffatmosphäre.

Ferner können Verbindungen der Formel I, in denen der Rest "Py" durch Halogen, beispielsweise durch Chlor oder Brom, substituiert ist, dehalogeniert werden. Die Dehalogenierung kann mit reduzierend wirkenden Substanzen, insbesondere mit katalytisch erregtem Wasserstoff oder nascierendem Wasserstoff erfolgen. Der Austausch des Halogens erfolgt beispielsweise durch Wasserstoff in Gegenwart von Raney-Nickel in beispielsweise alkoholischer Lösung, in Gegenwart von Platin in Eisessig oder vorzugsweise in Gegenwart von Palladium auf Kohle in wässriger Lösung bei Verwendung eines Salze der zu enthalogenisierenden Verbindung oder in einem für die Reaktion inerten Lösungsmittel.

Als Lösungsmittel können beispielsweise Aether, wie z.B. Tetrahydrofuran oder Dioxan, niedere Alkanole, wie z.B. Methanol oder Aethanol oder auch Lösungsmittelgemische, wie z.B. Methanol/Formamid verwendet werden. Die Reaktion wird beispielsweise bei Raumtemperatur, kann jedoch auch bei leicht erhöhter Temperatur, beispielsweise bei einer Temperatur bis zu 60°C und leichtem Ueberdruck ausgeführt werden. Die Enthalogenierung kann jedoch auch mit nascierendem Wasserstoff, beispielsweise mit Zink, insbesondere mit Zinkstaub oder auch metallischem Kupfer ausgeführt werden. Ferner können auch Natriumamalgam und Natriummethylat oder auch -äthylat in alkoholischer Lösung verwendet werden.

Verbindungen der Formel I, in denen der Rest "Py" zweifach durch Halogen, beispielsweise durch Chlor oder Brom in 2- und 6-Stellung substituiert sind, lassen sich durch Umsetzung mit einem gegebenenfalls substituierten Amin in Verbindungen der Formel I überführen, in denen ein Halogenatom in 2-Stellung durch ein gegebenenfalls substituiertes Amino ersetzt worden ist.

Die Umsetzungen mit einem gegebenenfalls substituierten Amin werden beispielsweise in alkoholischer Lösung bei erhähter Temperatur, vorzugsweise Rückflusstemperatur des Reaktionsgemisches und gegebenenfalls bei Ueberdruck ausgeführt.

Verbindungen der Formel I, in denen der Rest "Py" zweifach durch Halogen in 2- und 6-Stellung substituiert sind, lassen sich aus entsprechenden 2-Halogen-6-hydroxy oder -6-niederalkoxyverbindungen durch Umsetzung mit einem Halogenierungsmittel, beispielsweise einem Chlorierungsmittel wie Phosphoroxychlorid herstellen.

Sind im Rest "Py" als Substituenten Niederalkoxygruppen, wie z.B. Methoxy- oder Aethoxygruppen oder auch Phenoxygruppen vorhanden, so lassen sich diese leicht durch sauere oder basische Hydrolyse in Verbindung der Formel I umwandeln, in denen der Rest "Py" durch Hydroxy substituiert ist. Die neuen Ausgangsstoffe und Verfahren

zur Herstellung sind ebenfalls Gegenstand der Erfindung,

Je nach Verfahrensbedingungen und Ausgangsstoffen erhält man die Verbindungen der Formel I in freier Form oder in Form ihrer Salze, die sich in üblicher Weise ineinander oder in andere Salze umwandeln lassen. Säureadditionssalze lassen sich z.B. durch Umsetzen einer freien Verbindung der Formel I mit einer Säure, insbesondere einer organischen oder anorganischen Säure, die sich zur Bildung von pharmazeutisch verwendbaren Salzen eignet, erhalten. Als solche Säuren seien beispielsweise genannt: Halogenwasserstoffsäuren, Schwefelsäuren, Phosphorsäuren, Salpetersäure, Perchlorsäure, aliphatische, alicyclische, aromatische oder heterocyclische Carbon- oder Sulfonsäuren, wie Ameisen-, Essig-, Propion-, Bernstein-, Glykol-, Milch-, Aepfel-, Wein-, Zitronen-, Malein-, Hydroxymalein- oder Brenztraubensäure; Phenylessig-, Benzoe-, p-Aminobenzoe-, Anthranil-, p-Hydroxybenzoe-, Salicyl- oder p-Aminosalicylsäure, Embonsäure, Methansulfon-, Aethansulfon-, Hydroxyäthansulfon-, Aethylensulfonsäure; Halogenbenzolsulfon-, Toluolsulfon-, Naphthalinsulfonsäure oder Sulfanilsäure; oder Ascorbinsäure; Methionin, Trypthophan, Lysin oder Arginin. Säureadditionssalze von Verbindungen der Formel I können z.B. durch Behandeln mit alkalischen Mitteln, wie Alkalimetallhydroxiden, oder basischen Ionenaustauschern in die freien Basen oder z.B. durch Behandeln mit geeigneten Ionenaustauschern oder Silbersalzen in andere Salze übergeführt werden.

Die Erfindung betrifft auch diejenigen Ausführungsformen des Verfahrens, nach denen man von einer, auf irgendeiner Stufe des Verfahrens als Zwischenprodukt erhältlichen Verbindung ausgeht und die fehlenden Verfahrensschritte durchführt, oder das Verfahren auf irgendeiner Stufe abbricht, oder bei denen man ein Ausgangsmaterial unter den Reaktionsbedingungen bildet oder eine Reaktionskomponente gegebenenfalls in Form eines Derivates, z.B. eines Salzes, einsetzt.

Erhaltene Isomerengemische können nach an sich bekannten Methoden, z.B. durch fraktionierte Destillation, Kristallisation und/oder Chromatographie, in die einzelnen Isomeren getrennt werden.

Zweckmässig verwendet man für die Durchführung der erfindungsgemässen Verfahren diejenigen Ausgangsstoffe, die zu den eingangs besonders erwähnten Gruppen von Endstoffen und besonders zu den speziell beschriebenen oder hervorgehobenen Endstoffen führen.

Die vorliegende Erfindung betrifft zudem die Verbindungen der Formel I und deren pharmazeutisch verwendbare, nicht-toxische Säureadditionssalze zur Verwendung als Medikamente, insbesondere als Antihypertensiva, z.B. zur Behandlung von erhöhtem Blutdruck, sowie ihre Verwendung zur Herstellung von pharmazeutischen, insbesondere antihypertensiv wirksamen Präparaten.

Die vorliegende Erfindung betrifft ebenfalls pharmazeutische Präparate, welche Verbindungen der Formel I oder pharmazeutisch verwendbare Säureadditionssalze von solchen Verbindungen enthalten. Bei den erfindungsgemässen pharmazeutischen Präparaten handelt es sich um solche zur enteralen, wie oralen oder rektalen, sowie parenteralen Verabreichung, die den pharmakologischen Wirkstoff allein oder zusammen mit einem pharmazeutisch anwendbaren Trägermaterial enthalten.

Die neuen pharmazeutischen Präparate enthalten von etwa 10% bis etwa 95%, vorzugsweise von etwa 20% bis etwa 90% des Wirkstoffes. Erfindungsgemässe pharmazeutische Präparate in Dosiseinheitsformen sind z.B. Dragées, Tabletten, Kapseln, Suppositorien oder Ampullen. Die pharmazeutischen Präparate der vorliegenden Erfindung werden in an sich bekannter Weise, z.B. mittels konventioneller Misch-, Granulier-, Dragier-, Lösungs- oder Lyophilisierungsverfahren hergestellt.

So kann man pharmazeutische Präparate zur oralen Anwendung erhalten, indem man den Wirkstoff mit festen Trägerstoffen und gegebenenfalls Hilfsstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, hergestellt z.B. unter Verwendung von Mais-, Weizen-, Reis- oder Kartoffelstärke, Gelatine, Traganth, Methylcellulose, Hydroxypropyl-methylcellulose, Natriumcarboxymethylcellulose und/oder Polyvinylpyrrolidon, sowie gegebenenfalls Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrolidon, Polyäthylenglycol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffe, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffs mit einer Grundmasse enthalten; als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffes in wasserlöslicher Form, z.B. eines wasserlöslichen Salzes, ferner Suspensionen des Wirkstoffes, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat oder Triglyceride, verwendet, oder wässrige Injektionssuspensionen, welche viskositätserhöhende Stoffe, z.B. Natrium-carboxymethylcellulose, Sorbit und/oder Dextran, und gegebenenfalls auch Stabilisatoren enthalten.

Die Erfindung umfasst ebenfalls die Verwendung der Verbindungen der Formel I oder von pharmazeutisch verwendbaren nicht-toxischen Salzen von solchen Verbindungen als pharmakologisch wirksame Stoffe, insbesondere als Antihypertensiva, vorzugsweise in Form von pharmazeutischen Präparaten. Die Dosierung des Wirkstoffes hängt vom Warmblüter-Spezies, dem Körpergewicht und Alter und vom individuellen Zustand, sowie von der Applikationsweise ab. Durchschnittlich wird einem Warmblüter von etwa 70 kg Körpergewicht eine Tagesdosis von etwa 25 bis etwa 400 mg, vorzugsweise von etwa 50 bis etwa 200 mg Wirkstoff verabreicht.

Die nachfolgenden Beispiele illustrieren die oben beschriebene Erfindung; sie sollen jedoch diese in ihrem Umfang in keiner Weise einschränken. Temperaturen werden in Celsiusgraden angegeben.

Beispiel 1: Eine Suspension von 37.4 g des Hydrojodids von N-(2,6-Dimethyl-4-pyrimidinyl)-S-methyl-isothioharnstoff in 200 ml Methanol wird mit 7.6 g Aethylendiamin versetzt und auf dem Wasserbad erhitzt. Nach 5 Minuten erhält man eine klare Lösung, die während einer Stunde unter Rückfluss gekocht wird. Das Lösungsmittel wird mit Hilfe eines Rotationsverdampfers unter vermindertem Druck entfernt und der Rückstand während 2 Stunden bei 150° erhitzt. Nach Beendigung der Methylmercaptan-Abspaltung wird das Gemisch abgekühlt, in Diäthyläther aufgeschlämmt und dann filtriert. Der Filterrückstand wird in 50 ml heissem tert.-Butanol suspendiert und einige Zeit stehen gelassen. Man filtriert, wäscht mit Isopropanol nach und erhält so das Hydrojodid des 2-[(2,6-Dimethyl-4-pyrimidinyl)-amino]-2-imidazolins, F. 269-271°.

Das so erhältliche Salz wird in 200 ml Wasser gelöst, die Lösung mit 2-n wässriger Natriumhydroxidlösung alkalisch gestellt und das kristalline Material abfiltriert. Man löst die freie Base in Chloroform, die Lösung wird über Magnesiumsulfat getrocknet und eingedampft. Man erhält als Rückstand das 2-[(2,6-Dimethyl-4-pyrimidinyl)-amino]-2-imidazolin in Form von weissen Kristallen, F. 229-230°. Eine weitere Menge des Produkts kann durch Extrahieren der wässrigen Mutterlauge mit Chloroform erhalten werden.

Das Hydrochlorid von 2-[(2,6-Dimethyl-4-pyrimidinyl)-amino]-2-imidazolin kann erhalten werden, indem man eine Lösung der freien Verbindung in Isopropanol mit der berechneten Menge einer 1.9-n Lösung von Chlorwasserstoff in Aethanol behandelt. Das kristalline Produkt schmilzt bei 298-300°.

Das Ausgangsmaterial kann wie folgt hergestellt werden: Eine Suspension von 12.3 g 4-Amino-2,6-dimethyl-pyrimidin in 50 ml Chloroform wird tropfenweise mit 13.1 g Aethoxycarbonyl-isothiocyanat behandelt, und das Gemisch während einer Stunde unter Rückfluss erhitzt. Beim Abkühlen kristallisiert der N-(2,6-Dimethyl-4-pyrimidinyl)-N'-äthoxycarbonyl-thioharnstoff aus und wird aus 90%-igem wässrigem Aethanol umkristallisiert; er schmilzt bei 163-165°.

12.7 g des so erhältlichen N-(2,6-Dimethyl-4-pyrimidinyl)-N'-äthoxycarbonyl-thioharnstoffs wird mit 70 ml einer 1-n wässrigen Natriumhydroxid-Lösung versetzt, und das Gemisch während einer Stunde gekocht. Beim Abkühlen fällt der N-(2,6-Dimethyl-4-pyrimidinyl)-thioharnstoff in Form von weissen Kristallen aus, F. 236-238°.

Ein Gemisch von 20.4 g N-(2,6-Dimethyl-4-pyrimidinyl)-thioharnstoff und 130 ml Methanol wird mit 16.7 g Methyljodid behandelt und während einer Stunde unter Rückfluss gekocht, wobei das Ausgangsmaterial in Lösung geht. Das Lösungsmittel wird unter vermindertem Druck verdampft, und man erhält als Rückstand das Hydrojodid des N-(2,6-Dimethyl-4-pyrimidinyl)-S-methyl-isothioharnstoffs in Form von weissen Kristallen, F. 200-205°; das Produkt wird ohne Reinigung weiterverarbeitet.

Beispiel 2: Eine Suspension von 35.7 g des Hydrojodids von N-(2,6-Dimethoxy-4-pyrimidinyl)-S-methyl-isothioharnstoff in 750 ml Methanol wird tropfenweise mit 12.0 g Aethylendiamin in 100 ml Methanol versetzt und während 6 Stunden unter Rückfluss gekocht. Nach Beendigung der Methylmercaptan- und Ammoniakentwicklung wird das Reaktionsgemisch unter vermindertem Druck auf ein Volumen von etwa 100 ml eingeengt und während 16 Stunden gekühlt. Der kristalline Niederschlag wird abfiltriert und mit Wasser, dann mit Essigsäureäthylester gewaschen; man erhält so das 2-[(2,6-Dimethoxy-4-pyrimidinyl)-amino]-2-imidazolin, F. 197-200°.

Eine Suspension von 20 g 2-[(2,6-Dimethoxy-4-pyrimidinyl)-amino]-2-imidazolin und 200 ml Aethanol wird mit 39.6 ml einer 2,26-n Lösung von Chlorwasserstoff in Aethanol versetzt. Die so erhältliche Lösung wird nach dem Filtrieren unter vermindertem Druck eingeengt und portionenweise mit Essigsäureäthylester versetzt, wobei Kristallisation einsetzt. Das kristalline Material wird abfiltriert und ergibt das Hydrochlorid des 2-[(2,6-Dimethoxy-4-pyrimidinyl)-amino]-2-imidazolins, F. 193-195°.

Das Ausgangsmaterial kann wie folgt erhalten werden:
Ein Gemisch von 29.0 g 4-Amino-2,6-dimethoxy-pyrimidin und 24.5 g Aethoxycarbonyl-isothiocyanat in 100 ml Chloroform wird während 2 Stunden unter Rückfluss gekocht, dann abgekühlt, und unter vermindertem Druck eingedampft, und der Rückstand in 200 ml heissem 95%-igem wässrigem Aethanol suspendiert. Man kühlt ab, filtriert den Niederschlag ab und wäscht mit einem Gemisch von Aethanol und Essigsäureäthylester nach. Der N-(2,6-Dimethoxy-4-pyrimidinyl)-N'-äthoxycarbonyl-thioharnstoff wird in Form von gelben Kristallen erhalten, F. 177-180°.

Ein Gemisch von 14.3 g N-(2,6-Dimethoxy-4-pyrimidinyl)-N'-äthoxycarbonyl-thioharnstoff und 90 ml einer 1-n wässrigen Natriumhydroxidlösung wird während 90 Minuten unter Rückfluss gekocht, wobei das Ausgangsmaterial zunächst in Lösung geht und das kristalline Produkt dann ausfällt. Dieses wird abfiltriert, mit Wasser gewaschen und in 100 ml eines heissen 1:1-Gemisches von Isopropanol und Petroläther suspendiert. Der N-(2,6-Dimethoxy-4-pyrimidinyl)-thioharnstoff wird in Form von gelben Kristallen erhalten, F. 237-238°.

Eine Suspension von 24.4 g N-(2,6-Dimethoxy-4-pyrimidinyl)-thioharnstoff in 2500 ml Aceton wird tropfenweise mit 64.8 g Methyljodid behandelt und unter Rühren während einer Stunde unter Rückfluss gekocht. Man erhält nach kurzer Zeit eine klare Lösung, die sich kurz danach wieder trübt und ein kristallines Material abscheidet. Dieses wird abfiltriert und ergibt das Hydrojodid des N-(2,6-Dimethoxy-4-pyrimidinyl)-S-methyl-isothioharnstoffs, F. 185-187°, das ohne weitere Reinigung verwendet wird.

Beispiel 3: 12,3 g 4-Amino-2,6-dimethyl-pyrimidin und 18,3 g des Hydrojodids von 2-Methylthio-2-imidazolin werden pulverisiert und gut durchgemischt, dann auf eine Badtemperatur von 190° erhitzt, bei welcher Temperatur die Methylmercaptanabspaltung einsetzt. Die Badtemperatur wird auf 170° gesenkt; man belässt während 1 Stunde bei dieser Temperatur, kühlt dann auf 50° und löst die warme Schmelze in einem 1:1-Gemisch von Aceton und Methanol. Man filtriert und dampft das

Filtrat unter vermindertem Druck zur Trockne ein. Der Rückstand wird in einem Gemisch von Isopropanol und Aceton aufgenommen und filtriert; der Rückstand stellt das Hydrojodid des 2-[(2,6-Dimethyl-4-pyrimidinyl)-amino]-2-imidazolins dar und wird zwischen 2-n wässriger Natriumhydroxidlösung und Methylenchlorid verteilt. Die organische Phase wird abgetrennt, über Magnesiumsulfat getrocknet und eingedampft. Man erhält als halbkristallinen Rückstand das 2-[(2,6-Dimethyl-4-pyrimidinyl)-amino]-2-imidazolin, das in Wasser aufgenommen und in kristalliner Form abfiltriert wird, F. 225-229°. Das Produkt zeigt keine Mischschmelzpunkt-Depression mit einer Probe des nach dem Verfahren des Beispiels 1 erhältlichen Materials und ist laut Dünnschichtchromatogramm (System: Methanol) mit diesem identisch.

Eine weitere Menge der freien Verbindung kann aus der Isopropanol/Aceton-Mutterlauge erhalten werden, indem man diese unter vermindertem Druck eindampft, den Rückstand in Wasser löst, die wässrige Lösung mit 2-n. wässriger Natriumhydroxidlösung alkalisch stellt und mit Methylenchlorid extrahiert. Der organische Extrakt wird eingedampft, der Rückstand in Wasser gelöst, und die Lösung mit einer kleinen Menge 2-n. wässriger Natriumhydroxidlösung versetzt und gekühlt. Der kristalline Niederschlag wird abfiltriert; er schmilzt bei 227-229°.

Beispiel 4: Eine Lösung von 11 g des Hydrojodids von N-(4,6-Dimethoxy-2-methyl-5-pyrimidinyl)-S-methyl-isothioharnstoff und 2,1 g Aethylendiamin in 100 ml absolutem Aethanol wird während 10 Stunden unter Rückfluss gekocht. Anschliessend dampft man unter vermindertem Druck ein, verrührt den Rückstand gut mit einer gesättigten wässrigen Natriumcarbonatlösung und filtriert den Niederschlag ab. Nach Umkristallisieren aus Dimethylformamid erhält man das 2-[(4,6-Dimethoxy-2-methyl-5-pyrimidinyl)-amino]-2-imidazolin, F. 229°.

Das Ausgangsmaterial wird wie folgt hergestellt:
Eine Lösung von 4,2 g Ammonium-thiocyanat in 25 ml Aceton wird mit 6 ml Benzoylchlorid versetzt; das Gemisch wird kurz aufgekocht und

dann tropfenweise mit einer Lösung von 8,5 g 5-Amino-4,6-dimethoxy-2-methyl-pyrimidin in 45 ml Aceton behandelt. Anschliessend kocht man noch während 15 Minuten unter Rückfluss und giesst dann die Reaktionslösung in 500 ml Wasser. Das ausgefallene Produkt wird abfiltriert und gut mit Wasser gewaschen, dann in einer heissen Lösung von 8 g Natriumhydroxid in 80 ml Wasser aufgenommen. Man kocht während 5 Minuten unter Rückfluss, kühlt und stellt den pH-Wert durch Zugabe von 2-n. Salzsäure auf 9, worauf der N-(4,6-Dimethoxy-2-methyl-5-pyrimidinyl)-thioharnstoff auskristallisiert, der nach Umkristallisieren aus einem Gemisch aus Wasser und Dioxan bei 214-215° schmilzt.

Ein Gemisch von 8,9 g N-(4,6-Dimethoxy-2-methyl-5-pyrimidinyl)-thioharnstoff und 8,5 g Methyljodid in 800 ml Methanol wird während 3 Stunden unter Rückfluss gekocht und unter vermindertem Druck stark eingeengt. Das Hydrojodid des N-(4,6-Dimethoxy-2-methyl-5-pyrimidinyl)-S-methyl-isothioharnstoffs kristallisiert dabei aus, F. 168-170°, und wird ohne Reinigung weiterverarbeitet.

Beispiel 5: Ein Gemisch von 40,0 g des Hydrojodids von N-(2,6-Dimethyl-4-pyrimidinyl)-S-methyl-isothioharnstoff und 18,3 g Propylendiamin werden in 200 ml Methanol während 6 Stunden unter Rückfluss gekocht. Man kühlt ab, filtriert und verdampft die klare Lösung unter vermindertem Druck zur Trockne. Der Rückstand wird in 100 ml Wasser aufgenommen und dreimal mit je 150 ml Chloroform extrahiert. Der organische Extrakt wird über Magnesiumsulfat getrocknet und eingedampft. Man erhält so das 2-[(2,6-Dimethyl-4-pyrimidinyl)-amino]-1,4,5,6-tetrahydro-pyrimidin, F. 161-163°, das wie folgt in das Hydrochlorid übergeführt wird:

Eine in der Wärme bereitete Lösung von 12 g 2-[(2,6-Dimethyl-4-pyrimidinyl)-amino]-1,4,5,6-tetrahydropyrimidin in 80 ml Isopropanol wird mit 42,7 ml einer 2,7-n Lösung von Chlorwasserstoff in Aethanol versetzt. Die Lösung wird unter vermindertem Druck auf etwa ein Drittel des Volumens eingeengt und portionenweise mit Essigsäureäthylester verdünnt. Der kristalline Niederschlag wird abfiltriert und ergibt das Dihydrochlorid des 2-[(2,6-Dimethyl-4-pyrimidinyl)-amino]-1,4,5,6-tetrahydro-pyrimidins, F. 255-258°.

Beispiel 6: Zu einer Lösung von 7,2 g Aethylendiamin in 30 ml Methanol tropft man eine Lösung von 20,6 g N-(6-Chlor-2-methyl-4-pyrimidinyl)-S-methyl-isothioharnstoff-hydrojodid in 300 ml Methanol. Die Innentemperatur beträgt 65° und wird während 6 Stunden aufrechterhalten. Nach beendeter Entwicklung von Methylmercaptan und Ammoniak wird am Rotationsverdampfer abgedampft und der Rückstand in 150 ml Wasser suspendiert, abgesaugt und mit Wasser gewaschen. Die Rohbase schmilzt bei 207-209°.

Das Hydrochlorid wird erhalten, indem man 16,6 g der Rohbase in 200 ml heissem Aethanol suspendiert und mit 34,1 ml 2,3-n äthanolischer Salzsäure versetzt, die erhaltene klare Lösung mit Aktivkohle filtriert, auf ca. 80 ml am Rotationsverdampfer eindampft und mit Essigester und wenig Aether das Hydrochlorid des 2-[(6-Chlor-2-methyl-4-pyrimidinyl)-amino]-2-imidazolins zur Kristallisation bringt, F. 299-301° (Zers.).

Der als Ausgangsstoff dienende Isothioharnstoff wird wie folgt erhalten:

a) 65,2 g 4,6-Dichlor-2-methyl-pyrimidin werden in 750 ml Aethanol mit 100 g flüssigem Ammoniak in einem Autoklaven 12 Stunden bei 80° gerührt, das ausgeschiedene Ammoniumchlorid wird abfiltriert, am Rotationsverdampfer eingedampft, der Rückstand in 1 Liter Wasser heiss suspendiert und abgesaugt. Aus der wässrigen Mutterlauge werden durch Verdampfen auf ca. 100 ml weitere Mengen des 4-Amino-6-chlor-2-methyl-pyrimidins gewonnen. Die vereinigten Rohprodukte werden aus Essigester umgelöst, F. 185-187°.

b) 28,7 g des gereinigten Produktes werden in 200 ml Chloroform und 50 ml Dimethylformamid gelöst, zur Lösung werden 26,2 g Aethoxycarbonyl-isothiocyanat zugegeben und das Gemisch 2 Stunden auf 80° erwärmt. Man kühlt, verdampft und kristallisiert den Rückstand, den $N^1$-(6-Chlor-2-methyl-4-pyrimidinyl)-$N^3$-äthoxycarbonyl-thioharnstoff

aus Aethanol um, F. 142-143°.

c) Durch Verseifung mit 1-n. Natronlauge (1 Stunde, 130°) wird der N-(6-Chlor-2-methyl-4-pyrimidinyl)-thioharnstoff vom F. 230° (Verfärbung und Sintern) erhalten.

d) 13,5 g dieses Thioharnstoffs werden in 1500 ml Aceton suspendiert, mit 37,8 g Methyljodid versetzt und 2 Stunden unter Rückfluss gekocht. Nach 15 Minuten tritt Lösung ein. Das Lösungsmittel wird am Rotationsverdampfer eingedampft, die zurückbleibenden Kristalle in Aether suspendiert und isoliert, F. 179° (Zers.). Es handelt sich um das als Ausgangsmaterial genannte Isothioharnstoff-hydrojodid.

Beispiel 7: 10,6 g der nach Beispiel 6 erhaltenen 2-[(6-Chlor-2-methyl-4-pyrimidinyl)-amino]-2-imidazolin-Base werden in 200 ml Wasser und 50 ml 1-n. Salzsäure gelöst, mit 1 g 10%-igem Palladium-auf-Kohle versetzt und unter leichtem (0,2 bar) Ueberdruck mit Wasserstoff bei 48° geschüttelt. Nach Aufnahme der berechneten Menge (1120 ml) wird unterbrochen, filtriert, am Rotationsverdampfer eingedampft und der kristalline Rückstand in Aethanol-Methanol 1:1 heiss umkristallisiert. Die erhaltenen Kristalle des 2-[(2-Methyl-4-pyrimidinyl)-amino]-2-imidazolin-dihydrochlorids schmelzen bei 252-255°.

Beispiel 8: 24,0 g N-(6-Chlor-2-methoxy-4-pyrimidinyl)-S-methyl-isothioharnstoff-hydrojodid werden in 300 ml Methanol gelöst und zu 8,0 g Aethylendiamin in 75 ml Methanol zugetropft. Man erhitzt 6 Stunden unter Rühren bei Rückflusstemperatur, wobei Kristallisation eintritt, engt am Rotationsverdampfer ein und suspendiert den Rückstand in 200 ml Wasser. Anschliessend wird isoliert und mit Wasser gewaschen. Man erhält so das 2-[(6-Chlor-2-methoxy-4-pyrimidinyl)-amino]-2-imidazolin vom Schmelzpunkt 216-219°.

Hydrochlorid: 11,5 g Base werden in 50 ml Methanol suspendiert, und 1 Aequivalent 2,4-n äthanolischer Salzsäure zugegeben. Man verdünnt mit 50 ml Aethanol, filtriert mit Celite und verdampft die klare Mutterlauge am Rotationsverdampfer auf die Hälfte. Nach Zugabe von 100 ml Aceton fällt das Hydrochlorid des 2-[(6-Chlor-2-methoxy-4-pyrimidinyl)-amino]-2-imidazolins vom F. 220° kristallin aus.

Der als Ausgangsmaterial verwendete Isothioharnstoff wird analog zum Beispiel 6 gewonnen:

a) Aus 63,8 g 4-Amino-6-chlor-2-methoxy-pyrimidin (hergestellt aus 2,6-Dichlor-4-amino-pyrimidin und Natriummethanolat in Methanol bei 80° Aussentemperatur) und 52,4 g Aethoxycarbonyl-isothiocyanat in siedendem Aceton wird der $N^1$-(6-Chlor-2-methoxy-4-pyrimidinyl)-$N^3$-äthoxycarbonyl-thioharnstoff vom F. 160-164° erhalten.

b) Die Verseifung dieses Esters mit 150 ml 1-n Natronlauge in der Siedehitze ergibt einen kristallinen Niederschlag. Lösung und Niederschlag werden mit 10 ml Eisessig schwach sauer gestellt, wobei unter Schäumen die Decarboxylierung eintritt. Man isoliert den N-(6-Chlor-2-methoxy-4-pyrimidinyl)-thioharnstoff, F. über 330° und wäscht ihn mit Wasser.

c) Der so erhaltene Harnstoff wird wie in Beispiel 6 mit Methyljodid in das als Ausgangsmaterial genannte Isothioharnstoff-hydrojodid vom F. 167° (Zers.) übergeführt.

<u>Beispiel 9:</u> 11,5 g des nach Beispiel 8 erhaltenen 2-[(6-Chlor-2-methoxy-4-pyrimidinyl)-amino]-2-imidazolins werden in 200 ml Methanol und 100 ml Dimethylformamid (purissimum) suspendiert und 2 g 10%-iger Palladium-auf-Kohle-Katalysator zugegeben und bei 0,2 bar Ueberdruck und einer Temperatur von 49° mit Wasserstoff behandelt. Die Wasserstoffaufnahme verläuft langsamer als in Beispiel 7. Nach ihrer Beendigung wird der Katalysator filtriert, die Mutterlauge am Rotationsverdampfer eingedampft, der Rückstand mit 100 ml 1-n Natronlauge versetzt und mit Chloroform ausgezogen. Der Chloroform-Rückstand besteht aus einer langsam kristallisierenden Base, die direkt in ihr Hydrochlorid übergeführt wird, indem sie in 50 ml Aceton mit 1 Aequivalent äthanolischer Salzsäure versetzt, dann durch Zugabe von Aceton und

Essigester kristallin abgeschieden wird. Das erhaltene 2-[(2-Methoxy-4-pyrimidinyl)-amino]-2-imidazolin-hydrochlorid schmilzt bei 170-172° (Zers.).

Beispiel 10: 22,75 g des nach Beispiel 8 erhaltenen 2-[(6-Chlor-2-methoxy-4-pyrimidinyl)-amino]-imidazolins werden in 100 ml 1-n wässriger Salzsäure und 300 ml Wasser gelöst, mit 2 g 10%-iger Palladium-auf-Kohle versetzt und bei 0,2 bar Ueberdruck und 60° Innentemperatur hydriert. Nach Aufnahme von 2320 ml (ber. 2240 ml) wird unterbrochen, vom Katalysator abgenutscht und am Rotationsverdampfer völlig eingedampft. Der Rückstand wird zweimal mit 100 ml Alkohol abgedampft, die Kristalle in 150 ml Isopropanol heiss suspendiert und erneut isoliert. Infolge Spaltung der Methoxygruppe ist das 2-[(2-Hydroxy-4-pyrimidinyl)-amino]-2-imidazolin-hydrochlorid entstanden und weist einen F. von 256-258° (Zers.) auf.

Beispiel 11: 28,0 g N-(6-Chlor-2-dimethylamino-4-pyrimidinyl)-S-methyl-isothioharnstoff-hydrojodid werden in 400 ml Methanol gelöst und zu einer Lösung von 9,0 g Aethylendiamin in 75 ml Methanol zugetropft. Man rührt 6 Stunden bei Rückflusstemperatur, wobei die Ammoniak- und Methylmercaptan-Entwicklung zum Stillstand kommt und Kristalle abgeschieden werden. Man engt die Suspension im Rotationsverdampfer auf ca. 75 ml ein, saugt ab, verrührt den kristallinen Anteil mit 150 ml Wasser, isoliert ihn und wäscht mit Wasser und Isopropanol. Das erhaltene 2-[(6-Chlor-2-dimethylamino-4-pyrimidinyl)-amino]-2-imidazolin schmilzt bei 268-271°. Nach Umkristallisation aus Dimethylsulfoxid-Methanol schmilzt das Produkt bei 278-279°.

Die erhaltene Base wird mit der berechneten Mengen 2-n.wässriger Salzsäure versetzt, wobei das Hydrochlorid teilweise kristallisiert. Durch Zugabe der 4-fachen Menge Wasser wird bei 75° eine klare Lösung erhalten, welche nach Filtration am Rotationsverdampfer verdampft, der Rückstand in Isopropanol suspendiert, abgesaugt und mit Aethylacetat gewaschen wird. Das Hydrochlorid schmilzt bei 279-280°.

Zu einer Suspension von 7,8 g Base in 300 ml Wasser werden bei Raumtemperatur 3,42 g Methansulfonsäure zugegeben, wobei eine klare Lösung entsteht. Man dampft zur Trockene ein und kristallisiert den

Rückstand aus Methanol-Aether um. Das so erhaltene Methansulfonat schmilzt bei 256-259°.

Der als Ausgangsmaterial verwendete Isothioharnstoff wird analog zu Beispiel 6 gewonnen:

a) Aus 4-Amino-2,6-dichlor-pyrimidin wird mit Dimethylamin in Methanol in exothermer Reaktion das 4-Amino-6-chlor-2-dimethylamino-pyrimidin vom F. 151-152° hergestellt. 34,5 g dieser Verbindung werden mit 26,2 g Aethoxycarbonyl-isothiocyanat in 150 ml Aceton unter Rückfluss umgesetzt zum $N^1$-(6-Chlor-2-dimethylamino-4-pyrimidinyl)-$N^3$-äthoxycarbonyl-thioharnstoff vom F. 206-208°.

b) Die Verseifung dieser Verbindung mit 170 ml 1 n Natronlauge bei Rückflusstemperatur liefert einen dicken Kristallbrei, der abgesaugt und mit Wasser gründlich gewaschen wird. Der erhaltene N-(6-Chlor-2-dimethylamino-4-pyrimidinyl)-thioharnstoff schmilzt bei 235° (Zers.).

c) 29,0 g dieses Thioharnstoffs werden in 2,9 Liter Aceton suspendiert und mit 71 g Methyljodid versetzt. Beim Erhitzen unter Rückfluss geht die Suspension in Lösung und kristallisiert kurz danach wieder aus. Man kühlt ab und isoliert die kristalline Fällung des N-(6-Chlor-2-dimethylamino-4-pyrimidinyl)-S-methyl-isothioharnstoff-hydrojodids vom F. 220° (Zers.).

<u>Beispiel 12</u>: 11,1 g 2-[(6-Chlor-2-dimethylamino-4-pyrimidinyl)-amino]-2-imidazolin-Base (hergestellt nach Beispiel 11) werden mit 19,5 ml 1-n. Salzsäure und 80 ml Wasser gelöst, mit 2 g 10%-igem Palladium-auf-Kohle bei 0,2 bar Ueberdruck am Wasserstoff hydriert. Die Temperatur beträgt 52-55°. Nach 15 Stunden ist die berechnete Menge Wasserstoff (438 ml) aufgenommen. Man filtriert vom Katalysator, engt die wässrige Mutterlauge am Rotationsverdampfer ein, löst den Rückstand in 75 ml Isopropanol und lässt kristallisieren. Das erhaltene 2-[(2-Dimethylamino-4-pyrimidinyl)-amino]-2-imidazolin-dihydrochlorid schmilzt bei 275-278° (Zers.).

Beispiel 13: In gleicher Weise wie in Beispiel 11 beschrieben, wird aus 11,5 g Aethylendiamin in 100 ml Methanol, zu dem 38,0 g N-(6-Chlor-2-diäthylamino-4-pyrimidinyl)-S-methyl-isothioharnstoff - hydrojodid in 200 ml Methanol zugetropft werden, nach 6 Stunden Rühren unter Rückfluss die 2-[(6-Chlor-2-diäthylamino-4-pyrimidinyl)-amino]-2-imidazolin-Base vom F. 231-232° erhalten. Durch Suspendieren von 21,5 g der Base in 50 ml Methanol und Zugabe von 2 Aequivalenten (67 ml) 2,39-n äthanolischer Salzsäure, gefolgt von 300 ml Essigester, wird das Dihydrochlorid erhalten, welches beim Trocknen im Hochvakuum in das Monohydrochlorid vom F. 203-205 übergeht.

Das Ausgangsmaterial für die oben beschriebene Synthese lässt sich in Analogie zu Beispiel 11 erhalten:

a) aus 4-Amino-2,6-dichlor-pyrimidin mit Diäthylaminüberschuss in Methanol bei 80° Aussentemperatur das 4-Amino-6-chlor-2-diäthylamino-pyrimidin vom F. 110°, welches als Rohprodukt weiterverwendet wird;

b) durch Umsetzung von 43,6 g dieses Pyrimidins mit 28,5 g Aethoxy-carbonyl-isothiocyanat in siedendem Aceton der $N^1$-(6-Chlor-2-diäthyl-amino-4-pyrimidinyl)-$N^3$-äthoxycarbonyl-thioharnstoff vom F. 141-145°;

c) durch Verseifung von 48,0 g des erhaltenen Esters mit 250 ml 1-n Natronlauge unter Rückfluss der N-(6-Chlor-2-diäthylamino-4-pyrimidinyl)-thioharnstoff vom F. 175-180°; und

d) aus diesem rohen Thioharnstoff (35,8 g) mit 40 g Methyljodid in 750 ml Aceton unter Rückfluss das als Ausgangsprodukt verwendete N-(6-Chlor-2-diäthylamino-4-pyrimidinyl)-S-methyl-isothioharnstoff-hydrojodid vom F. 178-180° (Zers.).

Beispiel 14: In gleicher Weise wie in den Beispielen 11 und 13 beschrieben, werden aus N-(6-Chlor-2-di-n-butylamino-4-pyrimidinyl)-S-methyl-isothioharnstoff-hydrojodid und Aethylendiamin in Methanol das 2-[(6-Chlor-2-di-n-butylamino-4-pyrimidinyl)-amino]-2-imidazolin und sein Hydrochlorid erhalten. Die freie Base schmilzt bei 167-168°, das Hydrochlorid bei 148-150°.

Das Ausgangsmaterial für dieses Dibutylaminoderivat wird in gleicher Reaktionsfolge wie in den Beispielen 11 und 13 aus 4-Amino-2-di-n-butylamino-6-chlorpyrimidin erhalten.

Beispiel 15: 10,3 g Aethylendiamin werden in 40 ml Methanol vorgelegt und unter Rühren bei 70° 30,3 g N-(6-Dimethylamino-2-methyl-4-pyrimidinyl)-S-methyl-isothioharnstoff-hydrojodid in 350 ml Methanol zugetropft und das Reaktionsgemisch 6 Stunden bei dieser Temperatur gehalten. Das Lösungsmittel mit den suspendierten Kristallen wird im Rotationsverdampfer zur Trockne gebracht und der Rückstand in 300 ml Wasser suspendiert. Eine Probe zeigt, dass die Kristalle noch geringe Mengen Hydrojodid enthalten. Sie werden daher in 200 ml 2-n Salzsäure gelöst und mit 5-n Natronlauge gefällt. Die kristalline Base wird isoliert, zweimal mit warmem Wasser gewaschen, dann mit Isopropanol und Aether behandelt. Man erhält so das analysenreine 2-[(6-Dimethylamino-2-methyl-4-pyrimidinyl)-amino]-2-imidazolin, F. 286-288°.

Sein Hydrochlorid wird erhalten, indem die Base in Isopropanol suspendiert, mit 1 Aequivalent äthanolischer 2,3-n Salzsäure versetzt und mit Essigester als Monohydrochlorid gefällt wird, F. 281-283°.

Das Ausgangsmaterial für die oben beschriebene Synthese lässt sich analog zu den Beispielen 11 und 13 erhalten:

a) aus dem in Beispiel 6 gebrauchten 4-Amino-6-chlor-2-methyl-pyrimidin vom Schmelzpunkt 185-187° und der 4fachen theoretischen Menge Dimethylamin in Methanol bei 120° im Druckgefäss das 4-Amino-6-dimethylamino-2-methylpyrimidin vom F. 176-179°;

b) durch Umsetzung von 29,0 g dieses Pyrimidins mit 25,0 g Aethoxycarbonyl-isothiocyanat in siedendem Chloroform der $N^1$-(6-Dimethylamino-2-methyl-4-pyrimidinyl)-$N^3$-äthoxycarbonyl-thioharnstoff vom F. 140-143° (nach Umkristallisieren aus Alkohol);

c) durch Verseifen von 31,0 g des erhaltenen Esters mit 200 ml 1-n Natronlauge bei Siedetemperatur der N-(6-Dimethylamino-2-methyl-4-pyrimidinyl)-thioharnstoff vom F. 245-247°; und

d) aus 19,3 g dieses erhaltenen Thioharnstoffs mit 51,9 g Methyljodid in 2000 ml Aceton und 200 ml Methanol unter Rückfluss das N-(6-Dimethylamino-2-methyl-4-pyrimidinyl)-S-methyl-isothioharnstoff-hydrojodid vom F. 225-227°.

Beispiel 16: Zu einer siedenden Lösung von 9,5 g Aethylendiamin in 50 ml Methanol werden 26,0 g N-(2,6-Dihydroxy-5-pyrimidinyl)-S-methyl-isothioharnstoff-hydrojodid in 500 ml warmem Methanol zugetropft und 15 Stunden unter Rückfluss gekocht. Nach kurzer Zeit beginnt ein weisser Niederschlag auszufallen. Man kühlt, saugt ab und behandelt den Niederschlag, der neben der Imidazolin-Base schwerlösliches Hydrojodid enthält, nebst nicht umgesetzten Isothioharnstoff-hydrojodid, mehrmals 12 Stunden lang mit 50° warmem Wasser, dem 10% Methanol zugesetzt worden sind. Der Schmelzpunkt steigt dabei von 268° (Zers.) auf 290° (Zers.) und bleibt schliesslich bei 328-330° konstant. Der letztere Schmelzpunkt entspricht dem reinen 2-[(2,6-Dihydroxy-5-pyrimidinyl)-amino]-2-imidazolin, das zum grössten Teil in seiner tautomeren Form, dem 2-[(2,6-Dioxo-1,2,3,6-tetrahydro-5-pyrimidinyl)-amino]-2-imidazolin vorliegt.

Das Hydrochlorid wird durch Lösen von 7,5 g der Base in 38,5 ml 1-n Salzsäure in der Wärme erhalten. Die Lösung wird filtriert, am Rotationsverdampfer eingedampft, der Rückstand mit 100 ml Aethanol abgedampft, zum Rückstand 50 ml Essigester und 50 ml Isopropanol zugegeben und gerührt bis Kristallisation eintritt. F. 275° (Zers.) Kristallwasserhaltige Hydrochloride (F. 130° Zers.) werden im Hochvakuum getrocknet bis der Schmelzpunkt von 275° erreicht ist.

Das Ausgangsmaterial für die oben beschriebene Synthese wird in Analogie zu den Beispielen 11, 13 und 15 auf folgende Weise erhalten:

a) 12,5 g 5-Aminouracil werden in 150 ml Dimethylsulfoxid suspendiert und 13,1 g Aethoxycarbonyl-isothiocyanat zugegeben. Man rührt 3 Stunden bei 60°, erhält eine klare Lösung, giesst diese in Wasser, saugt ab und erhält den $N^1$-(2,6-Dihydroxy-5-pyrimidinyl)-$N^3$-äthoxycarbonyl-thioharnstoff, der zur Reinigung in siedendem Aceton suspendiert wird. F. > 300°;

b) durch einstündige Verseifung dieser Verbindung (12,9 g) mit 150 ml 1-n Natronlauge bei Rückflusstemperatur, Kühlen, Versetzen mit total 50 ml Eisessig wird der kristalline N-(2,6-Dihydroxy-5-pyrimidinyl)-thioharnstoff vom F. > 300° erhalten, der zur Reinigung in heissem Isopropanol suspendiert wird;

c) durch Umsetzen von 27,9 g dieser Verbindung mit 21,3 g Methyljodid in 1800 ml Methanol und 1000 ml Dimethylformamid bei 80° wird das N-(2,6-Dihydroxy-5-pyrimidinyl)-S-methyl-isothioharnstoff-hydrojodid vom F. 245° (Zers.) erhalten.

Beispiel 17: Zu einer Lösung von 8,5 g Aethylendiamin in 80 ml Methanol wird die Lösung von 26,0 g N-(2,6-Diäthyl-5-methyl-4-pyrimidinyl)-S-methyl-isothioharnstoff-hydrojodid in 250 ml Methanol zugetropft. Man kocht 12 Stunden unter Rühren, verdampft am Rotationsverdampfer zur Trockne, löst in Methylenchlorid, wäscht zweimal mit Wasser, verdampft das Lösungsmittel erneut, suspendiert den kristallinen Rückstand in Aethanol und erhält weisse Kristalle der Base vom F. 128-131°, die in das Hydrochlorid des 2-[(2,6-Diäthyl-5-methyl-4-pyrimidinyl)-amino]-2-imidazolins übergeführt werden, indem man 13,8 g der Base in Aethanol löst, 32,0 ml 1,82-n äthanolische Salzsäure zugibt und das Hydrochlorid mit etwas Aether zur Kristallisation bringt. F. 190-192°.

Das Ausgangsmaterial für die oben beschriebene Synthese lässt sich analog zu den Beispielen 11, 13, 15 und 16 wie folgt erhalten:

a) Aus 33,0 g 4-Amino-2,6-diäthyl-5-methyl-pyrimidin ("Kyanäthin") in 200 ml Aceton und 50 ml Dimethylformamid und 26,2 g Aethoxycarbonyl-isothiocyanat wird nach 3 Stunden Rückfluss der $N^1$-(2,6-Diäthyl-5-methyl-4-pyrimidinyl)-$N^3$-äthoxycarbonyl-thioharnstoff vom F. 86-89° erhalten.

b) Aus 49,5 g dieser Verbindung durch Verseifen mit 100 ml 2-n Natronlauge unter Rückfluss, Neutralisieren mit Eisessig auf pH 5 wird der N-(2,6-Diäthyl-5-methyl-4-pyrimidinyl)-thioharnstoff vom F. 151-154° erhalten.

c) Aus 17,1 g dieser Verbindung und 43,2 g Methyljodid in 400 ml heissem Aceton erhält man das N-(2,6-Diäthyl-5-methyl-4-pyrimidinyl)-S-methyl-isothioharnstoff-hydrojodid, F. 180-182°.

Beispiel 18: 27,0 g N-(2-Phenyl-4-pyrimidinyl)-S-methyl-isothioharnstoff-hydrojodid in 300 ml Methanol werden zu 9,0 g Aethylendiamin in 100 ml Methanol zugetropft. Man erwärmt 6 Stunden unter Rühren auf 80°, verdampft am Rotationsverdampfer zur Trockne, suspendiert die kristalline Verbindung in Wasser, dann in Isopropanol-Aethergemisch und isoliert so das 2-[(2-Phenyl-4-pyrimidinyl)-amino]-2-imidazolin vom F. 230-232°.

Aus 15 g der Imidazolin-Base wird das Dihydrochlorid durch Suspendieren in Methanol (50 ml), Zugabe von 2 Aequivalenten 2,3-n äthanolischer Salzsäure (54,4 ml), Filtrieren der entstandenen Lösung und Zugabe von Essigester als Kristalle vom F. 255-257° erhalten.

Das oben verwendete Ausgangsmaterial wird über folgende Stufen erhalten:

a) aus 51,3 g 4-Amino-2-phenyl-pyrimidin und 40 g Aethoxycarbonyl-isothiocyanat in 400 ml siedendem Aceton der $N^1$-(2-Phenyl-4-pyrimidinyl)-$N^3$-äthoxycarbonyl-thioharnstoff vom F. 185-188°;

b) aus 66,4 g des Esters durch Verseifen mit 300 ml 1-n Natronlauge in der Siedehitze den N-(2-Phenyl-4-pyrimidinyl)-thioharnstoff vom F. 237° (Zers.);

c) aus 38,3 g dieses Thioharnstoffes und 100 g Methyljodid in 3, 8 Liter siedendem Aceton das N-(2-Phenyl-4-pyrimidinyl)-S-methyl-isothioharnstoff-hydrojodid vom F. 220° (Zers.)

Beispiel 19: 22,8 g N-(2-Phenyl-4-pyrimidinyl)-S-methyl-isothioharnstoff-hydrojodid (Ausgangsmaterial von Beispiel 18) und 9,4 g Propylendiamin (1,3-Diaminopropan) werden in 300 ml Methanol 6 Stunden unter Rühren und Rückfluss erwärmt. Man dampft im Rotationsverdampfer zur Trockne, verteilt den öligen Rückstand zwischen 300 ml Wasser und

300 ml Chloroform, trocknet und verdampft die organische Phase und bringt sie mit 50 ml Essigester und 25 ml Aether zur Kristallisation. Das erhaltene 2-[(2-Phenyl-4-pyrimidinyl)-amino]-1,4,5,6-tetrahydro-pyrimidin schmilzt bei 166-169°.

Aus dieser Base wird in Aceton mit 1 Aequivelant äthanolischer Salzsäure das Monohydrochlorid vom F. 255° (Sintern ab 246°) erhalten.

Beispiel 20: Eine Lösung von 44,1 g des Hydrojodids von N-(2-Methyl-5-pyrimidinyl)-S-methyl-isothioharnstoff und 17,6 g Aethylendiamin in 400 ml absolutem Aethanol wird während 12 Stunden unter Rückfluss gekocht. Nach Filtration und Eindampfen zur Trockne verteilt man den öligen Rückstand zwischen 400 ml Chloroform und ca. 325 ml einer halbgesättigten Sodalösung, trennt die Schichten und extrahiert nachträglich die wässrige Schicht mehrmals mit Chloroform. Die vereinigten Chloroform-Extrakte ergeben nach Trocknen und Einengen beim Versetzen mit Petroläther einen kristallinen Niederschlag des 2-[(2-Methyl-5-pyrimidinyl)-amino]-2-imidazolins vom F. 187-189°.

Eine durch Erwärmen hergestellte Lösung von 21,3 g der obigen Base in 200 ml Isopropanol und 12,2 g Methansulfonsäure wird langsam mit Aether und wenig Essigester versetzt, worauf allmählich Kristallisation eintritt. Das so erhaltene Methansulfonat des 2-[(2-Methyl-5-pyrimidinyl)-amino]-2-imidazolins schmilzt nach dem Umkristallisieren aus einem Gemisch von Methanol und Aceton bei 149-151°.

Das als Ausgangsstoff verwendete Hydrojodid des N-(2-Methyl-5-pyrimidinyl)-S-methyl-isothioharnstoffs kann wie folgt hergestellt werden:

Eine Lösung von 26,5 g 2-Methyl-5-amino-pyrimidin in 750 ml Chloroform wird bei Raumtemperatur tropfenweise mit ca. 42 g Aethoxycarbonyl-isothiocyanat versetzt und das Gemisch danach während 1 Stunde bei Raumtemperatur und 2 1/2 Stunden unter Rückfluss gerührt. Nach dem Eindampfen zur Trockne kristallisiert man den festen Rückstand aus siedendem absolutem Aethanol um. Man erhält so den N-(2-Methyl-5-pyrimidinyl)-N'-äthoxycarbonyl-thioharnstoff vom F. 183-185°.

53 g des so erhältlichen N-(2-Methyl-5-pyrimidinyl)-N'-äthoxycarbonylthioharnstoffs werden mit 390 ml einer 1-n wässrigen Natriumhydroxidlösung versetzt. Man kocht die entstandene Lösung während 3 Stunden unter Rückfluss, kühlt ab und stellt den pH-Wert durch Zugabe von 6-n Salzsäure auf ca. 8 ein, worauf der N-(2-Methyl-5-pyrimidinyl)-thioharnstoff auskristallisiert, welcher nach dem Umkristallisieren aus siedendem absolutem Aethanol bei 191-193° unter Zersetzung schmilzt.

Eine Suspension von 28,75 g des obigen N-(2-Methyl-5-pyrimidinyl)-thioharnstoffs und 113 g Methyljodid in 1400 ml Aceton wird zum Rückfluss erhitzt, wobei das Ausgangsmaterial in Lösung geht. Ca. 20 Minuten später fallen Kristalle aus. Man kocht noch 1 3/4 Stunde weiter, kühlt auf Raumtemperatur, nutscht das Hydrojodid des N-(2-Methyl-5-pyrimidinyl)-S-methyl-isothioharnstoffs ab und wäscht mit wenig Aceton und Aether nach. F. ca. 152° unter Zersetzung.

Aus der Mutterlauge kann beim Einengen im Vakuum eine weitere Menge des kristallinen Hydrojodids erhalten werden.

Beispiel 21: In analoger Weise erhält man:

a) aus dem $N^1$-(5-Pyrimidinyl)-$N^3$-äthoxycarbonyl-thioharnstoff, F. 191-193° das 2-[(5-Pyrimidinyl)-amino]-2-imidazolin vom Schmelzpunkt 232-234°; das Hydrochlorid schmilzt bei 241-243° (Zers.);

b) aus dem $N^1$-(2-n-Butyl-5-pyrimidinyl)-$N^3$-äthoxycarbonyl-thioharnstoff, F. 134-135° das 2-[(2-n-Butyl-5-pyrimidinyl)-amino]-2-imidazolin vom Schmelzpunkt 145-147°; das Methansulfat schmilzt bei 100-102°;

c) aus dem $N^1$-(2-Phenyl-5-pyrimidinyl)-$N^3$-äthoxycarbonyl-thioharnstoff, F. 195-198° das 2-[(2-Phenyl-5-pyrimidinyl)-amino]-2-imidazolin vom Schmelzpunkt 250-252°; das Hydrochlorid schmilzt bei 230-233°;

d) aus dem $N^1$-[(6-Chlor-2-(4-morpholino)-4-pyrimidinyl]-$N^3$-äthoxycarbonyl-thioharnstoff, F. 185-189° das 2-[(6-Chlor-2-(4-morpholino)-4-pyrimidinyl)-amino]-2-imidazolin vom Schmelzpunkt 250-252°; das Hydrochlorid schmilzt bei 274-275°.

Beispiel 22: Zu einer Suspension von 18 g N-(2-Dimethylamino-6-methyl-4-pyrimidinyl)-S-methyl-isothioharnstoff-hydrojodid in 180 ml Aethanol gibt man 6,12 g Aethylendiamin, wobei eine klare Lösung entsteht. Anschliessend wird während 6 Stunden am Rückfluss gekocht. Das schon während der Reaktion ausgefallene Reaktionsprodukt wird abgenutscht und mit Isopropanol und Aether gewaschen. Das so erhaltene 2-[(2-Dimethylamino-6-methyl-4-pyrimidinyl)-amino]-2-imidazolin schmilzt bei 280-283°.

Zu einer Suspension von 10,35 g dieser Base in 300 ml abs. Aethanol gibt man 4,6 g Methansulfonsäure und erwärmt auf dem Wasserbad, wobei eine klare Lösung entsteht. Man dampft zur Hälfte ein und gibt sukzessive 175 ml Aether zu, wobei sich das Methansulfonat vom F: 226-228° ausscheidet.

Der als Ausgangsstoff verwendete Isothioharnstoff wird analog zu Beispiel 6 gewonnen:

4-Amino-2-dimethylamino-6-methyl-pyrimidin werden mit 11,15 g Aethoxy-carbonyl-isothiocyanat in 200 ml abs. Tetrahydrofuran unter Rückfluss zum $N^1$-(2-Dimethylamino-6-methyl-4-pyrimidinyl)-$N^3$-äthoxy-carbonyl-thioharnstoff vom F: 199-201° umgesetzt.

Diese Verbindung wird mit 180 ml Natronlauge und 100 ml Aethanol während 2 Stunden am Rückfluss gekocht. Man engt im Vakuum ein und stellt mit 2-n. Salzsäure auf pH = 7,5 und nutscht den Kristallbrei ab. Nach Umkristallisieren aus siedendem Alkohol schmilzt der so erhaltene N-(2-Dimethylamino-6-methyl-4-pyrimidinyl)-thioharnstoff bei 241-243°.

10,9 g dieses Thioharnstoffs werden in 1,2 l Aceton suspendiert und mit 29,5 g Methyljodid versetzt. Beim Erhitzen unter Rückfluss geht die Suspension in Lösung und kristallisiert kurz danach wieder aus. Man kocht noch eine Stunde am Rückfluss und nutscht die kristalline

Fällung des N-(2-Dimethylamino-6-methyl-4-pyrimidinyl)-S-methyl-isothioharnstoff-hydrojodids vom F: 211-214° ab.

Beispiel 23: Zu einer Lösung von 2,1 g Aethylendiamin in 40 ml Methanol gibt man in einer Portion 6,4 g N-(2-Diäthylamino-5-pyrimidinyl)-S-methyl-isothioharnstoff-hydrojodid und kocht anschliessend während 6 Stunden am Rückfluss. Die gelbe Reaktionslösung wird unter vermindertem Druck eingedampft. Den Rückstand versetzt man mit 75 ml 2-n Natronlauge und schüttelt mit Petroläther zur Entfernung des 5-Amino-2-dimethylamino-pyrimidina aus. Die wässerig-ölige Schicht wird mehrmals mit Essigester extrahiert. Aus den eingedampften Essigesterauszügen erhält man nach Umkristallisieren aus Essigester das 2-[(2-Diäthylamino-5-pyrimidinyl)-amino]-2-imidazolin vom F: 169-171°.

1,1 g der so erhaltenen Base werden in 35 ml Isopropanol gelöst und mit 0,94 g Methansulfonsäure versetzt. Auf Zusatz von Aether kristallisiert das Di-methansulfonat vom F: 144-147° aus.

Der als Ausgangsmaterial verwendete Isothioharnstoff wird wie folgt hergestellt:

Zu einer Lösung von 12,4 g Natrium in 360 ml Aethanol gibt man 29,2 g N,N-Diäthyl-guanidin-hemisulfat und 28,5 g Malonsäure-diäthylester und kocht während 3 Stunden am Rückfluss. Anschliessend wird das Reaktionsgemisch eingedampft. Den Rückstand löst man in 400 ml Wasser und stellt mit konz. Salzsäure auf pH-3,5, worauf das 2-Diäthylamino-4,6-dihydroxy-pyrimidin vom F: 235-240°(Zersetzung) ausfällt.

Zu 25 ml rauchender Salpetersäure (d = 1,52) tropft man unter Rühren bei einer Innentemperatur von 15-18° innerhalb 25 Minuten 65 ml Eisessig. Anschliessend gibt man portionenweise unter gutem Rühren innerhalb 15 Minuten 21 g 2-Diäthylamino-4,6-dihydroxy-pyrimidin zu. Nach beendeter Zugabe wird noch 2 Stunden bei Raumtemperatur weitergerührt. Man giesst das Reaktionsgemisch auf Eis und nutscht das ausgefällene 2-Diäthylamino-4,6-dihydroxy-5-nitro-pyrimidin

vom F: 280-282° (Zersetzung) ab.

25,1 g 2-Diäthylamino-4,6-dihydroxy-5-nitro-pyrimidin werden in 150 ml Phosphoroxychlorid und 38 ml Diäthylanilin während 90 Minuten am Rückfluss gekocht. Die schwarze Reaktionslösung wird unter vermindertem Druck stark eingeengt. Den öligen Rückstand giesst man auf Eis und extrahiert mit Aether. Den aus den Aetherphasen erhaltenen Rückstand kristallisiert man aus Cyclohexan um. Man erhält so das 2-Diäthylamino-4,6-dichlor-5-nitro-pyrimidin vom F: 91-93°.

24,5 g 2-Diäthylamino-4,6-dichlor-5-nitro-pyrimidin werden in 250 ml Aethanol mit 12 g Raneynickel als Katalysator hydriert. Nach Aufnahme von 3 Moläquivalenten Wasserstoff wird vom Katalysator abfiltriert und das Filtrat mit 8-n alkoholischer Salzsäure sauer gestellt. Anschliessend engt man unter vermindertem Druck stark ein bis zu beginnenden Kristallisation. Man nutscht die Kristalle ab und erhält so das 2-Diäthylamino-4,6-dichlor-5-amino-pyrimidin-dihydrochlorid.

20,5 g 2-Diäthylamino-4,6-dichlor-5-amino-pyrimidin-dihydrochlorid werden in 600 ml Aethanol mit 2 g Palladium-auf-Kohle (5 %ig) als Katalysator und 23 g wasserfreiem Natriumacetat bei Raumtemperatur hydriert. Nach Aufnahme von 2 Moläquivalenten Wasserstoff filtriert man vom Katalysator und den ausgefallenen anorganischen Salzen ab und stellt das Filtrat mit überschüssiger 8-normaler alkoholischer Salzsäure stark sauer und dampft unter vermindertem Druck zur Trockene ein. Der ölige Rückstand, der das 2-Diäthylamino-5-amino-pyrimidin-dihydrochlorid enthält, wird in 250 ml Chloroform mit 55 g Aethoxycarbonyl-isothiocyanat und 25 ml Triäthylamin während 3 Stunden am Rückfluss gekocht. Die Reaktionslösung wird im Vakuum zur Trockne eingedampft. Den Rückstand chromatographiert man an Silicagel, indem man mit Toluol mit steigendem Essigesterzusatz eluiert. Man erhält so den $N^1$-(2-Diäthylamino-5-pyrimidinyl)-$N^3$-carbäthoxy-thioharnstoff vom F: 130-132°.

7,35 g $N^1$-(2-Diäthylamino-5-pyrimidinyl)-$N^3$-carbäthoxy-thioharn-

stoff werden mit 70 ml 1-n Natronlauge während 1 Stunde am Rückfluss gekocht. Nach dem Abkühlen wird das Reaktionsgemisch auf pH-8 gestellt. Man nutscht die ausgefallenen Kristalle ab und kristallisiert aus Essigester-Petroläther um.Man erhält so den N-(2-Diäthylamino-5-pyrimidinyl)-thioharnstoff vom F: 162-166°C.

4,05 g des obigen Thioharnstoffs werden in 25 ml Aceton mit 8,1 ml Methyljodid während 2 Stunden unter Rückfluss gerührt. Die Reaktionslösung wird eingedampft und der Rückstand aus Essigester unter Zusatz von Aether kristallisiert. Man erhält so das N-(2-Diäthylamino-5-pyrimidinyl)-S-methyl-isothioharnstoff-hydrojodid vom F: 175-177°.

Beispiel 24: Ein Gemisch von 6,6 g 2-[(6-Chlor-2-dimethylamino-5-methyl-4-pyrimidinyl)-amino]-2-imidazolin-hydrochlorid, 200 ml Essigsäure und 30,25 ml 1,5-normaler Salzsäure in Eisessig, wird unter Zusatz von 1,3 g eines 10 %igen Palladium-Kohle-Katalysators bis zur Aufnahme von 1 Moläquivalent Wasserstoff, bei 35°C und 4 bar Druck hydriert. Danach wird vom Katalysator abfiltriert und das Filtrat unter vermindertem Druck eingedampft. Nach Umkristallisation des Rückstandes aus Aethylalkohol, unter Zusatz von Aktivkohle erhält man als farbloses Kristallisat, das 2-[2-Dimethylamino-5-methyl-4-pyrimidinyl)-amino]-2-imidazolin-hydrochlorid, welches bei 263° (Zersetzung) schmilzt.

Der Ausgangsstoff wird gemäss dem Beispiel 29 hergestellt.

Beispiel 25: 5,26 g N-(6-Chlor-2-dimethylamino-4-pyrimidinyl)-dithiocarbaminsäure-methylester werden mit 1,3 g Aethylendiamin in 50 ml Acetonitril 8 Stunden am Rückfluss erhitzt. Dann wird das Lösungsmittel im Vakuum verdampft und der als Rückstand erhaltene 1-(6-Chlor-2-dimethylamino-4-pyrimidinyl)-3-(2-aminoäthyl)-thioharnstoff durch Verdampfen mit Toluol von noch anhaftender Feuchtigkeit befreit.

Das zurückbleibende dunkle Oel wird in 50 ml Diphenyläther 2 Stunden auf 200° erhitzt. Hierauf wird das Lösungsmittel im Kugelrohr am Vakuum abdestilliert, der halb-kristalline Rückstand mit Wasser verrührt, filtriert und das Produkt mit Wasser und Isopropanol gewaschen. Das erhaltene 2-[(6-Chlor-2-dimethylamino-4-pyrimidinyl)-amino]-2-imidazolin schmilzt bei 278-279°.

Der als Ausgangsstoff verwendete N-(6-Chlor-2-dimethylamino-4-pyrimidinyl)-dithiocarbaminsäure-methylester kann auf folgende Weise hergestellt werden:

8 g 4-Amino-5-chlor-2-dimethylamino-pyrimidin werden in 100 ml Dimethylformamid vorgelegt. Dazu werden portionenweise während 5 Minuten 2,4 g Natriumhydrid bei 0° zugegeben und 15 Minuten gerührt. Hierauf werden 1,5 ml Schwefelkohlenstoff bei 0-5° zugetropft und 30 Minuten nachgerührt. Es wird erneut bei 0-5° 1,2 g Natriumhydrid zugegeben, 15 Minuten gerührt und dann bei derselben Temperatur 0,75 ml Schwefelkohlenstoff zugegeben und 30 Minuten weiter gerührt. Nach erneuter Zugabe von 1,2 g Natriumhydrid und 0,75 ml Schwefelkohlenstoff in der oben beschriebenen Weise werden nach 30 Minuten bei 0-5° 2,8 ml Methyljodid zugegeben und die Reaktionslösung in 2 Stunden sich auf 20° erwärmen lassen. Dann wird mit Wasser zersetzt, unlösliches Material abfiltriert und das Filtrat mit 2-normaler Salzsäure auf pH 5-6 gestellt. Das ausgefallene Produkt wird mit Methylenchlorid ausgezogen, getrocknet und eingedampft, worauf der N-(6-Chlor-2-dimethylamino-4-pyrimidinyl)-dithiocarbaminsäure-methylester bei Zugabe von Aether auskristallisiert und abfiltriert wird. F: 150-151°.

Beispiel 26: 38 g 6-Chlor-2-dimethylamino-4-(dimethylthio-methylenimino)-pyrimidin und 9,2 ml Aethylendiamin in 400 ml Methanol werden 14 Stunden bei 60° gerührt. Das ausgefallene 2-[(6-Chlor-2-dimethylamino-4-pyrimidinyl)-amino]-2-imidazolin wird durch Filtration isoliert und schmilzt bei 278-279°.

Der Ausgangsstoff wird wie folgt hergestellt:

Eine Lösung von 70 g 4-Amino-6-chlor-2-dimethylamino-pyrimidin in 750 ml Dimethylformamid wird unter Stickstoffatmosphäre und Eis-Kochsalz-Kühlung portionenweise mit 19,8 g Natriumhydrid (50 % in Oel) versetzt, so dass die Temperatur 10° nicht übersteigt. Es wird noch eine Stunde im Eisbad gekühlt, bis die heftige Wasserstoffentwicklung aufhört. Danach werden 12,3 ml Schwefelkohlenstoff unter Kühlen bei 0-10° zugetropft und diese Temperatur für eine Stunde beibehalten. Nun wird nochmals 9,9 g Natriumhydrid in Portionen bei 0-10° zugegeben. Das Reaktionsgemisch wird 30 Minuten im Eisbad gerührt, wonach 6,1 ml Schwefelkohlenstoff bei 0-10° zugetropft werden. Es wird noch eine Stunde unter Eiskühlung nachgerührt, dann wird, wie oben beschrieben, nochmals mit 9,9 g Natriumhydrid und 6,1 ml Schwefelkohlenstoff behandelt. Nach Zugabe des Schwefelkohlenstoffs wird noch eine Stunde im Eisbad gerührt. Nun werden unter Eis-Kochsalz-Kühlung 63,4 ml Methyljodid innerhalb etwa 30 Minuten so zugetropft, dass die Temperatur 15° nicht übersteigt. Anschliessend wird das Kühlbad entfernt und noch 2 Stunden gerührt. Danach werden unter Eiskühlung langsam 200 ml Wasser zugetropft und das Reaktionsgemisch auf 2000 ml Wasser ausgegossen. Das ausgefallene Rohprodukt wird abgesaugt, gründlich mit Wasser gewaschen und danach dreimal mi je 1000 ml Cyclohexan ausgekocht. Die vereinigten Cyclohexanextrakte werden über Natriumsulfat getrocknet, filtriert und bis auf ein Volumen von etwa 150 ml eingeengt. Beim Kühlen im Eisbad kristallisiert farbloses 6-Chlor-2-dimethylamino-4-(dimethylthio-methylenimino)-pyrimidin, welches bei 85-87° schmilzt.

In gleicher Weise wird das 2-[(2,6-Dichlor-4-pyrimidinyl)-amino]-2-imidazolin (F: 215-217°) aus 4-Amino-2,6-dichlorpyrimidin erhalten. Das dabei als Zwischenprodukt auftretende 2,6-Dichlor-4-(dimethylthio-methylenimino)-pyrimidin schmilzt bei F. 99-100°.

<u>Beispiel 27</u>: 4,7 g 2-[(2,6-Dichlor-4-pyrimidinyl)-amino]-2-imidazolin und 7,8 ml alkoholische Dimethylamin-Lösung (33 %) werden in 30 ml Aethanol 90 Minuten unter Rückfluss gekocht. Das ausgefallene

2-[(6-Chlor-2-dimethylamino-4-pyrimidinyl)-amino]-2-imidazolin wird noch heiss abgenutscht und mit Wasser und Aethanol gewaschen. Das Produkt ist mit demjenigen des Beispiels 11 identisch.

Der Ausgangsstoff wird gemäss dem Beispiel 26 hergestellt.

Beispiel 28: Ein Gemisch von 1 g 6-Chlor-2-dimethylamino-4-cyanamino-pyrimidin und 0,5 ml Aethylendiamin wird mit einem Tropfen Schwefelkohlenstoff versetzt und 1 Stunde auf 100° erhitzt. Anschliessend engt man das Reaktionsgemisch am Rotavapor ein und suspendiert den Rückstand in 50 ml heissem Aethanol. Das unlösliche 2-[(6-Chlor-2-dimethylamino-4-pyrimidinyl)-amino]-2-imidazolin wird heiss abgenutscht und schmilzt bei 264-268°. Nach Umkristallisation aus Dimethylsulfoxid-Methanol steigt der Schmelzpunkt auf 278-279°.

Der Ausgangsstoff wird wie folgt hergestellt:

Eine Lösung von 6,3 g 4-Amino-6-chlor-2-dimethylamino-pyrimidin in 60 ml Dimethylformamid wird unter Stickstoffatomosphäre und Eis-Kochsalz-Kühlung portionenweise mit 1,75 g Natriumhydrid (50 % in Oel) versetzt, so dass die Temperatur 10° nicht übersteigt. Es wird noch 30 Minuten im Eisbad gekühlt, bis die heftige Wasserstoffentwicklung aufhört. Danach tropft man langsam eine Lösung von 3 g Bromcyan in 30 ml Dimethylformamid unter Eiskühlung zu, wobei die Temperatur 20° nicht übersteigen sollte. Nach erfolgter Zugabe rührt man noch 30 Minuten bei 0-10° und neutralisiert dann mit 2-normaler Salzsäure. Das Reaktionsgemisch wird auf 100 ml Eiswasser gegossen und mehrmals mit Essigester extrahiert. Die vereinigten Essigesterextrakte werden mit Natriumsulfat getrocknet und eingeengt. Durch Umkristallisation des Rückstandes aus Aethanol erhält man das kristalline 6-Chlor-2-dimethylamino-4-cyanamino-pyrimidin vom F. 229-232°.

Beispiel 29: 2,9 g (0,01 Mol) 6-Chlor-2-dimethylamino-4-(dimethylthiomethylenimino)-5-methyl-pyrimidin und 0,6 g (0,01 Mol) Aethylendiamin

werden in 50 ml absolutem Methylalkohol gelöst und 16 Stunden bei 60° gerührt. Danach wird auf Raumtemperatur abgekühlt und das ausgefallene 2-[(6-Chlor-2-dimethylamino-5-methyl-4-pyrimidinyl)-amino]-2-imidazolin durch Filtration isoliert. Das Kristallisat suspendiert man in Methylalkohol, säuert an mit 5-normaler methanolischer Salzsäure und dampft die klare Lösung unter vermindertem Druck ein. Nach Umkristallisation des Rückstandes aus Methylalkohol-Diäthyläther, unter Zusatz von Aktivkohle, erhält man als farbloses Kristallisat das 2-[(6-Chlor-2-dimethylamino-5-methyl-4-pyrimidinyl)-amino]-2-imidazolin-hydrochlorid, welches unter Zersetzung bei 300° schmilzt.

Der Ausgangsstoff wird wie folgt hergestellt:

Zu einer frischen Natriummethylatlösung, hergestellt aus 12,4 g (0,54 Mol) Natrium und 60 ml absolutem Methylalkohol, gibt man bei 60° Reaktionstemperatur, 36,8 g (0,27 Mol) N,N-Dimethylguanidin-hydrogensulfat, und kocht 20 Minuten unter Rückfluss. Danach wird bei Rückflusstemperatur innert 30 Minuten eine Lösung von 34,4 g (0,27 Mol) 2-Cyano-propionsäure-äthylester in 110 ml absolutem Methylalkohol zugetropft, und die weisse Suspension noch eine weitere Stunde gekocht. Nach Abkühlen des Gemisches, filtriert man ab, und stellt das Filtrat mit 4,5-normaler methanolischer Salzsäure auf pH 5-6 ein. Das ausgefallene Kristallisat wird abfiltriert und mit kaltem Methylalkohol und Diäthyläther gewaschen. Nach dem Trocknen im Hochvakuum bei 70°C erhält man als farbloses Kristallisat, das 4-Amino-2-dimethylamino-6-hydroxy-5-methyl-pyrimidin-hydrochlorid-monohydrat, vom F. 285-290°.

Ein Gemisch von 5,0 g (0,0244 Mol) 4-Amino-2-dimethylamino-6-hydroxy-5-methyl-pyrimidin-hydrochlorid, 22,3 ml (0,244 Mol) Phosphoroxychlorid und 2,55 ml (0,0183 Mol) Triäthylamin wird unter Rühren 9 Stunden am Rückfluss gekocht. Danach wird das überschüssige Phosphoroxychlorid unter vermindertem Druck abdestilliert, und das zurückbleibende viskose Oel auf Eiswasser ausgetragen. Reaktionstemperatur bis 50°. Nach Abklingen der Reaktion wird noch eine Stunde

bei 60°C gerührt und danach auf Raumtemperatur abgekühlt. Mit konz. Natronlauge wird auf pH 7 eingestellt, und eine weitere Stunde bei 55-60°C gerührt. Dabei muss der pH Wert durch mehrmaliges Zugeben von konz. Natronlauge korrigiert werden. Dann wird abgekühlt und viermal mit je 40 ml Chloroform extrahiert. Die vereinigten Chloroformextrakte werden mit Natriumsulfat getrocknet und unter vermindertem Druck eingedampft. Nach Umkristallisation des Rückstandes aus Isopropylalkohol, unter Zusatz von Aktivkohle, erhält man als gelbliches Kristallisat, das 4-Amino-6-chlor-2-dimethylamino-5-methyl-pyrimidin, vom F . 170-172°.

Eine Lösung von 2 g (0,0107 Mol) 4-Amino-6-chlor-2-dimethylamino-5-methyl-pyrimidin in 20 ml absolutem Dimethylformamid wird unter Stickstoffatmosphäre und Eis-Kochsalz-Kühlung mit 0,33 g (0,0069 Mol) Natriumhydrid (50 % in Oel) versetzt, und eine Stunde bei 0,5° gerührt. Danach werden 0,2 ml (0,0033 Mol) Schwefelkohlenstoff unter Kühlen bei 0,10°C zugegeben und diese Temperatur für eine Stunde beibehalten. Nun wird nochmals 0,33 g Natriumhydrid bei 0-5° zugegeben und 30 Minuten bei 0° gerührt, worauf man weitere 0,2 ml Schwefelkohlenstoff beigibt und das Gemisch eine Stunde unter Eiskühlung nachrührt. Dann wird, wie oben beschrieben, nochmals mit 0,34 g Natriumhydrid und 0,25 ml Schwefelkohlenstoff behandelt. Nach der Zugabe des Schwefelkohlenstoffs wird noch eine Stunde im Eisbad gerührt. Nun werden unter Eis-Kochsalz-Kühlung 1,65 ml (0,0267 Mol) Methyljodid während etwa 15 Minuten so zugetropft, dass die Temperatur 10° nicht übersteigt. Anschliessend wird das Kühlbad entfernt und noch zwei Stunden ausgerührt.

Danach giesst man das Reaktionsgemisch auf 50 ml Eiswasser und extrahiert dreimal mit je 30 ml Essigester. Die vereinigten Essigesterextrakte werden mit Natriumsulfat getrocknet und unter vermindertem Druck eingedampft. Durch Auskochen des Rückstandes mit Petroläther wird vom Mineralöl befreit. Zur Entfernung von noch anhaftender Feuchtigkeit und Spuren Dimethylformamid, wird mit Toluol behandelt,

und man erhält als öligen Rückstand das 6-Chlor-2-dimethylamino-4-(dimethylthio-methylenimino)-5-methyl-pyrimidin, welches direkt als Rohprodukt als Ausgangsstoff verwendet wird.

Gegebenenfalls kann aus Cyclohexan umkristallisiert werden, wobei man ein farbloses Kristallisat, welches bei 115-117° schmilzt, erhält.

Auf gleiche Weise werden auch die folgenden Verbindungen hergestellt: 2-[(2-Methylamino-4-pyrimidinyl)-amino]-2-imidazolin. Sein Dihydrochlorid schmilzt bei 275-280°.

2-[(6-Chlor-2-propylamino-4-pyrimidinyl)-amino]-2-imidazolin-hydrochlorid, F. 230-234°;

2-[(6-Chlor-2-(N-methyl-N-propyl-amino)-4-pyrimidinyl)-amino]-2-imidazolin-hydrochlorid, F. 187-189°;

2-[(2-Propylamino-4-pyrimidinyl)-amino]-2-imidazolin, F. 230-233°.

Das als Ausgangsstoff verwendete 4-Amino-6-chlor-2-propylamino-pyrimidin kann auf folgende Weise hergestellt werden:

Ein Gemisch von 12,3 g 4-Amino-2,6-dichlor-pyrimidin und 16,4 ml n-Propylamin in 700 ml Methylalkohol, wird unter Rühren zum Rückfluss erwärmt, und die bald klare Lösung weitere 16 Stunden gekocht. Danach wird unter vermindertem Druck zur Trockene eingedampft. Den Rückstand stellt man mit 2-normaler Natriumcarbonatlösung alkalisch und extrahiert mehrmals mit Chloroform. Die vereinigten Chloroformextrakte werden mit Natriumsulfat getrocknet und eingedampft. Nach Kristallisation des Rückstandes aus Petroläther filtriert man ab, und erhält auf diese Weise, das 4-Amino-6-chlor-2-propylamino-pyrimidin vom F. 85-90°.

Das als Ausgangsstoff verwendete 4-Amino-6-chlor-2-(N-methyl-N-propyl-amino)-pyrimidin kann auf die gleiche Weise wie das zuvor beschriebene 4-Amino-6-chlor-2-propylamino-pyrimidin hergestellt werden, indem man N-Methyl-N-propylamin anstatt n-Propylamin zur Reaktion

einsetzt. Das auf diese Art erhaltene 4-Amino-6-chlor-2-(N-methyl-N-propyl-amino)-pyrimidin wird als Rohprodukt zur folgenden Stufe angesetzt.

Das als Ausgangsstoff verwendete 4-Amino-2-propylamino-pyrimidin kann nach folgendem Verfahren hergestellt werden:

Ein Gemisch von 3,7 g 4-Amino-6-chlor-2-propylamino-pyrimidin, 140 ml Eisessig und 30 ml 2N Salzsäure wird unter Zusatz von 0,7 g eines 10 %igen Palladium-Kohle-Katalysators bis zur Aufnahme von 1 Moläquivalent Wasserstoff bei Raumtemperatur und 4 bar Druck hydriert. Danach wird vom Katalysator abfiltriert und das Filtrat unter vermindertem Druck eingedampft. Den Rückstand stellt man unter Zusatz von Eis, mit 2-normaler Natriumcarbonatlösung alkalisch und extrahiert mehrmals mit Chloroform. Die vereinigten Chloroformextrakte werden mit Natriumsulfat getrocknet und unter vermindertem Druck eingedampft. Das zurückbleibende 4-Amino-2-propylamino-pyrimidin wird ohne weitere Reinigung zur folgenden Stufe eingesetzt.

Beispiel 30: Analog zu den in den vorhergehenden Beispielen beschriebenen Methoden werden auch die folgenden Verbindungen hergestellt:

2-[(2,6-Diäthoxy-4-pyrimidinyl)-amino]-2-imidazolin, F. 193-195°;
2-[(2-Dimethylamino-6-methoxy-4-pyrimidinyl)-amino]-2-imidazolin, F. 90-92°;
2-[(2-Isopropoxy-6-methoxy-4-pyrimidinyl)-amino]-2-imidazolin, F. 209-210°;
2-[(2-Butoxy-6-methoxy-4-pyrimidinyl)-amino]-2-imidazolin, F. 184-185°;
2-[(6-Chlor-2-isopropoxy-4-pyrimidinyl)-amino]-2-imidazolin, F 210-211°;
2-[(2,6-Bis-dimethylamino-4-pyrimidinyl)-amino]-2-imidazolin, F. 310-312° und das
2-[(2-Isopropoxy-4-pyrimidinyl)-amino]-2-imidazolin. F. 261-263°.

Die Ausgangsstoffe werden wir folgt hergestellt:

11,5 g (0,5 Mol) Natrium werden in 400 ml absol. Alkohol gelöst, unter Rühren zum Kochen erhitzt und portionenweise in ca. 20 Minuten 32,8 g (0,2 Mol) 4-Amino-2,6-dichlor-pyrimidin zugegeben. Man kocht danach noch 8 Stunden, filtriert und dampft das Filtrat unter vermindertem Druck ein. Der Rückstand wird mit Wasser angerieben, filtriert und aus Alkohol umkristallisiert. Man erhält das 4-Amino-2,6-diäthoxy-pyrimidin, welches bei 106-108° schmilzt.

32,8 g (0,2 Mol) 4-Amino-2,6-dichlor-pyrimidin, 400 ml i-Propanol und eine Lösung von 4,6 g (0,2 Mol) Natrium in 100 ml i-Propanol werden 15 Stunden am Rückfluss gekocht. Man dampft unter vermindertem Druck ein, reibt den Rückstand mit Wasser an und erhält durch Filtrieren ein Rohprodukt, welches bei 108-112° schmilzt. Nach Umkristallisieren aus i-Propanol erhält man das 4-Amino-6-chlor-2-isopropoxy-pyrimidin, welches bei 125-127° schmilzt.

18,8 g (0,1 Mol) der letztgenannten Verbindung werden 8 Stunden mit einer Lösung von 2,3 g (0,1 Mol) Natrium in 100 ml Methanol gekocht. Man filtriert und dampf das Filtrat unter vermindertem Druck ein. Der Rückstand wird mit Wasser angerieben, filtriert und aus Methanol umkristallisiert. Man erhält das 4-Amino-2-isopropoxy-6-methoxy-pyrimidin, welches bei 89-90° schmilzt.

Anstelle der gereinigten Verbindung kann auch das oben beschriebene Rohprodukt, welches bei 108-112° schmilzt, verwendet werden.

In analoger Weise wird auch das 4-Amino-2-n-butoxy-6-methoxy-pyrimidin, welches bei 94-96° schmilzt, hergestellt.

Die erhaltenen 4-Aminoverbindungen können z.B. gemäss Beispiel 1 in die entsprechenden S-Methyl-isothioharnstoff-Verbindungen umgewandelt werden. Ihre Umsetzung mit Aethylendiamin ergibt die entsprechenden Imidazolin-Produkte.

Beispiel 31: Analog zu den in den vorhergehenden Beispielen, z.B. gemäss den Beispielen 11 und 13, werden auch die folgenden Verbindungen hergestellt:

2-[(2-Methyl-6-phenylamino-4-pyrimidinyl)-amino]-2-imidazolin. F. 263-265°. Das Hydrochlorid schmilzt bei 311-313°.

2-[(6-(4-Methoxy-phenyl)-amino-2-methyl-4-pyrimidinyl)-amino]-2-imidazolin. F. 266-269°. Das Hydrochlorid schmilzt bei 281-283°.

2-[(6-(4-Chlor-phenyl)-amino-2-methyl-4-pyrimidinyl)-amino]-2-imidazolin. F. 282-284°. Das Hydrochlorid schmilzt bei 320-322°.

2-[(2-Methyl-6-phenoxy-4-pyrimidinyl)-amino]-2-imidazolin. Das Hydrochlorid schmilzt bei 302-305°.

Die als Ausgangsstoffe verwendeten neuen Verbindungen und ihre neuen Vorstufen werden z.B. analog zu denjenigen der Beispiele 11 oder 13 wie folgt hergestellt:

Ad 1): Ausgehend von 4-Amino-6-chlor-2-methyl-pyrimidin und Anilin erhält man das 4-Amino-2-methyl-6-phenylamino-pyrimidin, F. 192-194°. Dieses ergibt nach Umsetzung mit Aethoxycarbonyl-isothiocyanat den entsprechenden Aethoxycarbonyl-thioharnstoff (F.199-200°), der mit 1-normaler Natriumhydroxydlösung zum entsprechenden Thioharnstoff verseift wird (F. 227-230°). Die Umsetzung dieses Thioharnstoffs mit Methyljodid ergibt den als Ausgangsstoff verwendeten N-(2-Methyl-6-phenylamino-4-pyrimidinyl)-S-methyl-isothioharnstoff-hydrojodid, welches bei ungefähr 150° schmilzt.

Ad 2): Ausgehend von 4-Amino-6-chlor-2-methyl-pyrimidin und p-Anisidin erhält man das 4-Amino-6-(4-methoxyphenyl)-amino-2-methyl-pyrimidin, F. 243-245°. Dieses ergibt nach Umsetzung mit Aethoxycarbonyl-isothiocyanat in Aceton und Dimethylformamid den entsprechenden Aethoxycarbonyl-thioharnstoff (F. 198-200°), der mit 1-normaler Natriumhydroxydlösung zum entsprechenden Thioharnstoff verseift wird. (F 221-224°). Die Umsetzung dieses Thioharnstoffes mit Methyljodid in Methanol-Dimethylformamid ergibt den als Ausgangsstoff verwendeten N-[ 6-(4-methoxy-phenyl)-amino-2-methyl-4-pyrimidinyl]-S-methyl-isothioharnstoff-hydrojodid, welches bei 165-167° schmilzt.

Ad 3): Ausgehend von 4-Amino-6-chlor-2-methyl-pyrimidin und p-Chloranilin erhält man das 4-Amino-6-(4-chlorphenyl)-amino-2-methyl-pyrimidin, F. 180-182°. Dieses ergibt nach Umsetzung mit Aethoxycarbonylisothiocyanat in Aceton und Dimethylformamid den entsprechenden Aethoxycarbonyl-thioharnstoff (F. 200-202°), der mit 2-normaler Natriumhydroxydlösung zum entsprechenden Thioharnstoff verseift wird (F. 242-245° unter Zersetzung). Die Umsetzung dieses Thioharnstoffes mit Methyljodid in Aceton ergibt den als Ausgangsstoff verwendeten N-[ 6-(4-chlorphenyl)-amino-2-methyl-4-pyrimidinyl]-S-methyl-isothioharnstoff-hydrojodid, welches bei 212-215° schmilzt.

Ad 4): Ausgehend von 4-Amino-6-chlor-2-methyl-pyrimidin und Phenolnatrium erhält man das 4-Amino-2-methyl-6-phenoxy-pyrimidin (F.165-167°). Dieses ergibt nach Umsetzung mit Aethoxycarbonyl-isothiocyanat in Aceton den entsprechenden Aethoxycarbonyl-thioharnstoff (F. 139-141°), der mit 1-normaler Natriumhydroxydlösung zum entsprechenden Thioharnstoff verseift wird (F. 221-224°). Die Umsetzung der letztgenannten Verbindung mit Methyljodid in Methanol-Dimethylformamid ergibt den als Ausgangsstoff verwendeten N-(2-Methyl-6-phenoxy-4-pyrimidinyl)-S-methyl-isothioharnstoff-hydrojodid.

Beispiel 32: Analog zu den in den vorhergehenden Beispielen beschriebenen Methoden werden auch die folgenden Verbindungen hergestellt:

2-[(4-Pyrimidinyl)-amino]-2-imidazolin.

2-[(2-Butylamino-4-pyrimidinyl)-amino]-2-imidazolin.

Beispiel 33: Tabletten enthaltend 0,1 g des Hydrochlorids von 2-[(2,6-Dimethyl-4-pyrimidinyl)-amino]-2-imidazolin, können wie folgt hergestellt werden:

Zusammensetzung (für 1000 Tabletten):

| | |
|---|---|
| 2-[(2,6-Dimethyl-4-pyrimidinyl)-amino]-2-imidazolin-hydrochlorid | 100,0 g |
| Lactose | 50,0 g |
| Weizenstärke | 73,0 g |
| Kolloidale Kieselsäure | 13,0 g |
| Magnesiumstearat | 2,0 g |
| Talk | 12,0 g |
| Wasser | q.s. |

Das 2-[(2,6-Dimethyl-4-pyrimidinyl)-amino]-2-imidazolin-hydrochlorid wird mit einem Teil der Weizenstärke, mit der Lactose und der kolloidalen Kieselsäure vermischt und das Gemisch durch ein Sieb getrieben. Ein weiterer Teil der Weizenstärke wird mit der 5-fachen Menge Wasser auf dem Wasserbad verkleistert und die obige Pulvermischung mit diesem Kleister angeknetet, bis eine schwach plastische Masse entsteht. Diese wird durch ein Sieb von 3 mm Maschenweite gedrückt, getrocknet und das trockene Granulat nochmals durch ein Sieb getrieben. Darauf werden die restliche Weizenstärke, der Talk und das Magnesiumstearat zugemischt und die erhaltene Mischung zu Tabletten von 0,25 g verpresst.

In analoger Weise werden auch Tabletten oder andere pharmazeutische Präparate, welche eine andere Verbindung der Erfindung, z.B. eine solche der vorhergehenden Beispiele enthalten, hergestellt.

Patentansprüche

(für alle benannten Länder ausser Oesterreich)

1. 2-(Pyrimidinylamino)-1,3-diaza-2-cycloalken-Verbindungen der allgemeinen Formel I

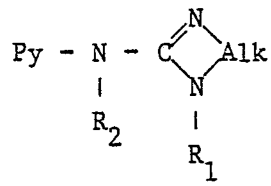

(I),

worin Py einen gegebenenfalls substituierten über ein Kohlenstoffatom mit dem Stickstoffatom verbundenen 4- oder 5-Pyrimidinylrest darstellt, $R_1$ und $R_2$ unabhängig voneinander Wasserstoff, Niederalkyl oder Niederalkenyl bedeuten, und Alk für Niederalkylen steht, das die beiden Stickstoffatome durch 2 bis 4 Kohlenstoffatome trennt, deren tautomere Verbindungen und Säureadditionssalze.

2. Verbindungen der im Anspruch 1 gezeigten Formel I, worin Py für gegebenenfalls durch einen, zwei oder drei gleiche oder verschiedene Substituenten der Gruppe Niederalkyl, Hydroxy, Niederalkoxy, Niederalkylthio, Halogen, Trifluormethyl, Niederalkylsulfonyl, Amino, gegebenenfalls durch Niederalkyl, Niederalkoxy, Hydroxy, Amino, Niederalkylamino, Diniederalkylamino oder Halogen substituiertes Phenyl, Phenoxy oder Phenylamino, Niederalkylamino, Dinieralkylamino, Pyrrolidino, Piperidino, Morpholino, Thiomorpholino, Niederalkanoylamino, Niederalkoxycarbonylamino, Ureido, 3-Niederalkylureido und 3,3-Diniederalkylureido substituiertes, über ein Kohlenstoffatom mit dem Stickstoffatom verbundenes 4- oder 5-Pyrimidinyl steht, $R_1$ und $R_2$ unabhängig voneinander Wasserstoff, Niederalkyl oder Niederalkenyl bedeuten, und Alk für Niederalkylen steht, das die beiden Stickstoffatome durch 2 bis 4 Kohlenstoffatome trennt, wobei mit "nieder" bezeichnete Reste bis zu 4 Kohlenstoffatomen enthalten, ihre tautomeren Verbindungen und Säureaddtionssalze.

3. Verbindungen der im Anspruch 1 gezeigten Formel I, worin Py für gegebenenfalls durch einen, zwei oder drei gleiche oder verschiedene

Substituenten der Gruppe Niederalkyl, Hydroxy, Niederalkoxy, Halogen, Amino, gegebenenfalls durch Niederalkyl, Niederalkoxy, Hydroxy, Amino, Niederalkylamino, Diniederalkylamino oder Halogen substituiertes Phenyl, Niederalkylamino, Diniederalkylamino, Pyrrolidino, Piperidino, Morpholino, Thiomorpholino, Niederalkanoylamino, Niederalkoxycarbonylamino, Ureido, 3-Niederalkylureido und 3,3-Diniederalkylureido substituiertes, über ein Kohlenstoffatom mit dem Stickstoffatom verbundenes 4- oder 5-Pyrimidinyl steht, $R_1$ und $R_2$ unabhängig voneinander Wasserstoff, Niederalkyl oder Niederalkenyl bedeuten, und Alk für Niederalkylen steht, das die beiden Stickstoffatome durch 2 bis 4 Kohlenstoffatome trennt, wobei mit "nieder" bezeichnete Reste bis zu 4 Kohlenstoffatomen enthalten, deren tautomere Verbindungen und Säureadditionssalze.

4. Verbindungen der im Anspruch 1 gezeigten Formel I, worin Py für gegebenenfalls durch einen, zwei oder drei gleiche oder verschiedene Substituenten der Gruppe Niederalkyl, Niederalkoxy, Phenyl, Amino, Niederalkylamino, Diniederalkylamino oder Morpholino und/oder Halogen substituiertes, über ein Kohlenstoffatom mit dem Stickstoffatom verbundenes 4- oder 5-Pyrimidinyl steht, $R_1$ Wasserstoff oder Niederalkyl und $R_2$ Wasserstoff oder Niederalkyl darstellen, und Alk für Niederalkylen steht, das die beiden Stickstoffatome durch 2 bis 3 Kohlenstoffatome trennt, wobei mit "nieder" bezeichnete Reste bis zu 4 Kohlenstoffatomen enthalten, und Halogen ein Atomgewicht bis zu 35 aufweist, und ihre Säureadditionssalze.

5. Verbindungen der allgemeinen Formel II

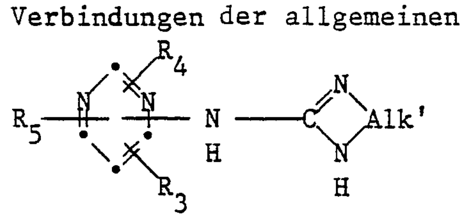

(II),

worin Alk' für Niederalkylen mit bis zu 4 Kohlenstoffatomen, das die beiden Stickstoffatome durch 2 bis 3 Kohlenstoffatome trennt, steht, und jeder der Reste $R_3$, $R_4$ und $R_5$ Wasserstoff, Niederalkyl mit bis zu

4 Kohlenstoffatomen oder Niederalkoxy mit bis zu 4 Kohlenstoffatomen, Halogen, Diniederalkylamino, Morpholino oder Phenyl, bedeutet, und ihre Säureadditionssalze.

6. Verbindungen der allgemeinen Formel III

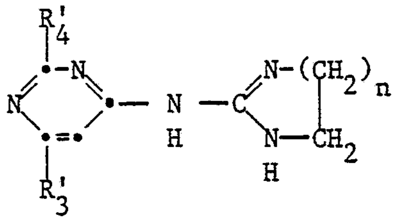

(III),

worin $R_3'$ und $R_4'$ unabhängig voneinander für Wasserstoff, Niederalkyl mit bis zu 4 Kohlenstoffatomen oder Niederalkoxy mit bis zu 4 Kohlenstoffatomen, Halogen oder Diniederalkylamino stehen, und n für 1 oder 2 steht, und ihre Säureaddtionssalze.

7. 2-[(6-Chlor-2-dimethylamino-4-pyrimidinyl)-amino]-2-imidazolin und seine Säureadditionssalze.

8. 2-[(6-Chlor-2-diäthylamino-4-pyrimidinyl)-amino]-2-imidazolin und seine Säureadditionssalze.

9. 2-[(6-Chlor-2-(4-morpholino)-4-pyrimidinyl)-amino]-2-imidazolin und seine Säureadditionssalze.

10. Hypotensiv und antihypertensiv wirksame Verbindungen gemäss Anspruch 1 , deren tautomere Verbindungen und ihre Säureadditionssalze.

11. Hypotensiv und antihypertensiv wirksame Verbindungen gemäss Anspruch 2, deren tautomere Verbindungen und ihre Säureadditionssalze.

12. Pharmazeutische Präparate enthaltend Verbindungen gemäss einem der Ansprüche 1 und 3 bis 10 oder therapeutisch verwendbare Säureadditionssalze von solchen Verbindungen.

13. Pharmazeutische Präparate enthaltend Verbindungen gemäss einem der Ansprüche 2 und 11 oder therapeutisch verwendbare Säureadditionssalze von solchen Verbindungen.

14. Die Verbindungen der Ansprüche 1 und 3 bis 10 zur Verwendung als Pharmazeutika.

15. Die Verbindungen der Ansprüche 2 und 11 zur Verwendung als Pharmazeutika.

16. Die Verbindungen der Ansprüche 1 und 3 bis 10 zur Anwendung in einem Verfahren zur therapeutischen Behandlung des menschlichen oder tierischen Körpers.

17. Die Verbindungen der Ansprüche 2 und 11 zur Anwendung in einem Verfahren zur therapeutischen Behandlung des menschlichen oder tierischen Körpers.

18. Verfahren zur Herstellung von neuen 2-(Pyrimidinylamino)-1,3-diaza-2-cycloalken-Verbindungen der allgemeinen Formel I

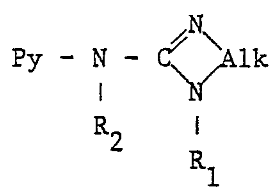

(I),

worin Py einen gegebenenfalls substituierten über ein Kohlenstoffatom mit dem Stickstoffatom verbundenen 4- oder 5-Pyrimidinylrest darstellt, $R_1$ und $R_2$ unabhängig voneinander Wasserstoff, Niederalkyl oder Niederalkenyl bedeuten, und Alk für Niederalkylen steht, das die beiden Stickstoffatome durch 2 bis 4 Kohlenstoffatome trennt, ihren tautomeren Verbindungen und Säureadditionssalzen davon, dadurch gekennzeichnet, dass man

a) eine Verbindung der Formel Py-X (IV) oder ein Salz davon mit einer Verbindung der Formel

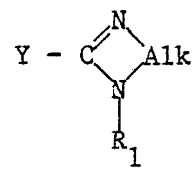

(V)

oder einem Salz davon umsetzt, worin einer der Reste X und Y eine Aminogruppe der Formel $-N(R_2)-H$ (VI) darstellt, und der andere eine unter den Reaktionsbedingungen zusammen mit Wasserstoff abspaltbare Gruppe bedeutet, oder

b) eine Verbindung der Formel VII

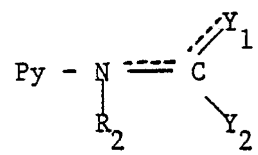

(VII),

worin $Y_1$ die Iminogruppe, eine abspaltbare Gruppe, die Oxo- oder Thioxogruppe bedeutet, und $Y_2$ eine abspaltbare Gruppe oder $Y_1$ und $Y_2$ zusammengenommen ein dreifach gebundenes Stickstoffatom bedeuten, wenn $R_2$ die Bedeutung von Wasserstoff hat, oder die entsprechende tautomere Form, oder ein Salz davon, mit einer Alkylendiamin-Verbindung der Formel $H_2N-Alk-NHR_1$ (VIII) umsetzt, oder

c) ein Phosphinsäurehalogenid der Formel XII

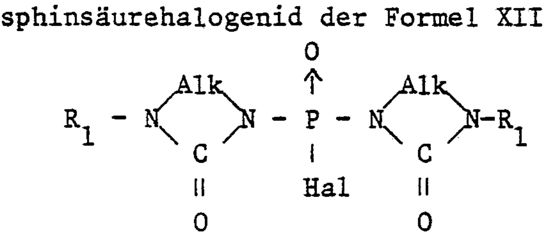

(XII),

worin Hal Halogen bedeutet, mit einer Amin-Verbindung der Formel $Py-NH-R_2$ (IVa) umsetzt, und, wenn erwünscht, eine erhaltene Verbindung der Formel I, in der der Rest Py Halogen enthält, enthalogeniert, oder eine erhaltene Verbindung der Formel I, worin der Rest Py in 2-und 6-Stellung durch Halogen substituiert ist, das Halogen in 2-Stellung durch gegebenenfalls substituiertes Amino austauscht, und/oder wenn erwünscht, eine erhaltene Verbindung in eine andere Verbindung der Erfindung umwandelt, und/oder, wenn erwünscht, eine erhaltene Base in ein Säureadditionssalz oder ein erhaltenes Salz in die freie Base oder in ein anderes Säureadditionssalz überführt, und/oder, wenn erwünscht, ein erhaltenes Gemisch von Isomeren in die einzelnen Isomeren auftrennt.

Patentansprüche
(für Oesterreich)

1. Verfahren zur Herstellung von neuen 2-(Pyrimidinylamino)-1,3-diaza-2-cycloalken-Verbindungen der allgemeinen Formel I

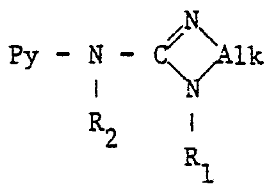

(I),

worin Py einen gegebenenfalls substituierten über ein Kohlenstoffatom mit dem Stickstoffatom verbundenen 4- oder 5-Pyrimidinylrest darstellt, $R_1$ und $R_2$ unabhängig voneinander Wasserstoff, Niederalkyl oder Niederalkenyl bedeuten, und Alk für Niederalkylen steht, das die beiden Stickstoffatome durch 2 bis 4 Kohlenstoffatome trennt, ihren tautomeren Verbindungen und Säureadditionssalzen davon, dadurch gekennzeichnet, dass man

a) eine Verbindung der Formel Py-X (IV) oder ein Salz davon mit einer Verbindung der Formel

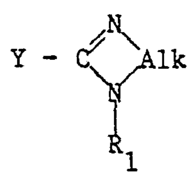

(V)

oder einem Salz davon umsetzt, worin einer der Reste X und Y eine Aminogruppe der Formel $-N(R_2)-H$ (VI) darstellt, und der andere eine unter den Reaktionsbedingungen zusammen mit Wasserstoff abspaltbare Gruppe bedeutet, oder

b) eine Verbindung der Formel VII

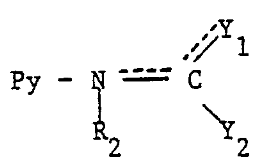

(VII),

worin $Y_1$ die Iminogruppe, eine abspaltbare Gruppe, die Oxo- oder Thioxogruppe bedeutet, und $Y_2$ eine abspaltbare Gruppe oder $Y_1$ und $Y_2$ zusammengenommen ein dreifach gebundenes Stickstoffatom bedeuten, wenn $R_2$ die

Bedeutung von Wasserstoff hat, oder die entsprechende tautomere Form, oder ein Salz davon, mit einer Alkylendiamin-Verbindung der Formel $H_2N$-Alk-$NHR_1$ (VIII) umsetzt, oder

c) ein Phosphinsäurehalogenid der Formel XII

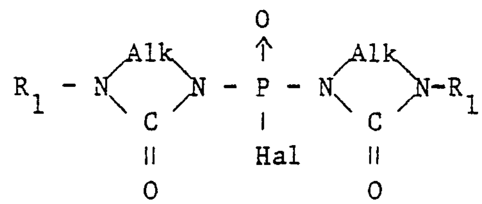

(XII),

worin Hal Halogen bedeutet, mit einer Amin-Verbindung der Formel Py-NH-$R_2$ (IVa) umsetzt, und, wenn erwünscht, eine erhaltene Verbindung der Formel I, in der der Rest Py Halogen enthält, enthalogeniert, oder eine erhaltene Verbindung der Formel I, worin der Rest Py in 2- und 6-Stellung durch Halogen substituiert ist, das Halogen in 2-Stellung durch gegebenenfalls substituiertes Amino austauscht, und/oder wenn erwünscht, eine erhaltene Verbindung in eine andere Verbindung der Erfindung umwandelt, und/oder, wenn erwünscht, eine erhaltene Base in ein Säureadditionssalz oder ein erhaltenes Salz in die freie Base oder in ein anderes Säureadditionssalz überführt, und/oder, wenn erwünscht, ein erhaltenes Gemisch von Isomeren in die einzelnen Isomeren auftrennt.

2. Verfahren nach Anspruch 1, dadurch gekennzeichnet, dass man in der Reaktion a) Ausgangsstoffe verwendet, in welchen eine zusammen mit Wasserstoff abspaltbare Gruppe X oder Y eine freie oder verätherte Mercaptogruppe, eine reaktionsfähige, funktionell abgewandelte Hydroxygruppe oder die Nitroaminogruppe ist.

3. Verfahren nach Anspruch 1, dadurch gekennzeichnet, dass man in der Reaktion b) Ausgangsstoffe verwendet, in welchen eine abspaltbare Gruppe $Y_1$ oder $Y_2$ eine freie oder verätherte Mercaptogruppe, eine reaktionsfähige, funktionell abgewandelte Hydroxygruppe oder die Nitroaminogruppe ist.

4. Verfahren nach Anspruch 1, dadurch gekennzeichnet, dass man in der Reaktion b) die Kondensation zum Ring in einer oder zwei Stufen durchführt.

5. Verfahren nach einem der Ansprüche 1 bis 4, dadurch gekennzeichnet, dass man Verbindungen der allgemeinen Formel I, worin Py für gegebenenfalls durch einen, zwei oder drei gleiche oder verschiedene Substituenten der Gruppe Niederalkyl, Hydroxy, Niederalkoxy, Niederalkylthio, Halogen, Trifluormethyl, Niederalkylsulfonyl, Amino, gegebenenfalls durch Niederalkyl, Niederalkoxy, Hydroxy, Amino, Niederalkylamino, Diniederalkylamino oder Halogen substituiertes Phenyl, Phenoxy oder Phenylamino, Niederalkylamino, Diniederalkylamino, Pyrrolidino, Piperidino, Morpholino, Thiomorpholino, Niederalkanoylamino, Niederalkoxycarbonylamino, Ureido, 3-Niederalkylureido und 3,3-Diniederalkylureido substituiertes, über ein Kohlenstoffatom mit dem Stickstoffatom verbundenes 4- oder 5-Pyrimidinyl steht, $R_1$ und $R_2$ unabhängig voneinander Wasserstoff, Niederalkyl oder Niederalkenyl bedeuten, und Alk für Niederalkylen steht, das die beiden Stickstoffatome durch 2 bis 4 Kohlenstoffatome trennt, wobei mit "nieder" bezeichnete Reste bis zu 4 Kohlenstoffatomen enthalten, ihre tautomeren Verbindungen und Säureadditionssalze herstellt.

6. Verfahren nach einem der Ansprüche 1 bis 4, dadurch gekennzeichnet, dass man Verbindungen der allgemeinen Formel I, worin Py für gegebenenfalls durch ein, zwei oder drei gleiche oder verschiedene Substituenten der Gruppe Niederalkyl, Niederalkoxy, Phenyl, Amino, Niederalkylamino, Diniederalkylamino oder Morpholino und/oder Halogen substituiertes, über ein Kohlenstoffatom mit dem Stickstoffatom verbundenes 4- oder 5-Pyrimidinyl steht, $R_1$ Wasserstoff oder Niederalkyl und $R_2$ Wasserstoff oder Niederalkyl darstellen, und Alk für Niederalkylen steht, das die beiden Stickstoffatome durch 2 bis 3 Kohlenstoffatome trennt, wobei mit "nieder" bezeichnete Reste bis zu 4 Kohlenstoffatomen enthalten, und Halogen ein Atomgewicht bis zu 35 aufweist, und ihre Säureadditionssalze herstellt.

7. Verfahren nach einem der Ansprüche 1 bis 4, dadurch gekennzeichnet, dass man Verbindungen der allgemeinen Formel II

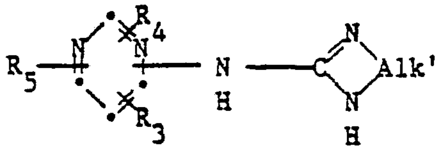

(II),

worin Alk' für Niederalkylen mit bis zu 4 Kohlenstoffatomen, das die beiden Stickstoffatome durch 2 bis 3 Kohlenstoffatome trennt, steht, und jeder der Reste $R_3$, $R_4$ und $R_5$ Wasserstoff, Niederalkyl mit bis zu 4 Kohlenstoffatomen oder Niederalkoxy mit bis zu 4 Kohlenstoffatomen, Halogen, Diniederalkylamino, Morpholino oder Phenyl, bedeutet, und ihre Säureadditionssalze herstellt.

8. Verfahren nach einem der Ansprüche 1 bis 4, dadurch gekennzeichnet, dass man Verbindungen der allgemeinen Formel III

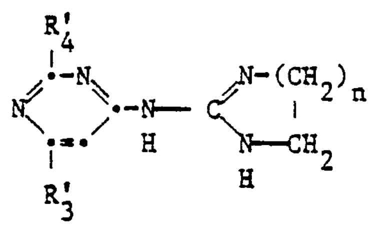

(III),

worin $R_3'$ und $R_4'$ unabhängig voneinander für Wasserstoff, Niederalkyl mit bis zu 4 Kohlenstoffatomen oder Niederalkoxy mit bis zu 4 Kohlenstoffatomen, Halogen oder Diniederalkylamino stehen, und n für 1 oder 2 steht, und ihre Säureadditionssalze herstellt.

9. Verfahren nach einem der Ansprüche 1 bis 4, dadurch gekennzeichnet, dass man 2-[(6-Chlor-2-dimethylamino-4-pyrimidinyl)-amino]-2-imidazolin und seine Säureadditionssalze herstellt.

10. Verfahren nach Anspruch 1 zur Herstellung von neuen 2-(Pyrimidinylamino)-1,3-diaza-2-cycloalken-Verbindungen der im Anspruch 1 gezeigten allgemeinen Formel I, worin alle Symbole die im Anspruch 1 angegebenen Bedeutungen haben, ihren tautomeren Verbindungen und Säureadditionssalzen davon, dadurch gekennzeichnet, dass man

a) eine Verbindung der Formel Py-X (IV) oder ein Salz davon mit einer Verbindung der im Anspruch 1 gezeigten Formel V oder einem Salz davon umsetzt, worin einer der Reste X und Y eine Aminogruppe der Formel $-N(R_2)-H$ (VI) darstellt, und der andere eine unter den Reaktionsbedingungen zusammen mit Wasserstoff abspaltbare Gruppe bedeutet, oder

$b_1$) eine Verbindung der Formel VIIa

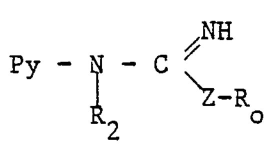

(VIIa),

worin Z für Sauerstoff oder Schwefel steht, und $R_o$ einen gegebenenfalls substituierten Kohlenwasserstoffrest bedeutet, oder ein Salz davon, mit einer Alkylendiamin-Verbindung der Formel $H_2N - Alk - NH - R_1$ (VIII) umsetzt, oder

$b_2$) eine Verbindung der allgemeinen Formel IXa

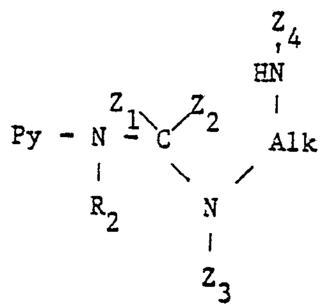

(IXa)

worin $Z_1$ und $Z_2$ zusammen für Sauerstoff oder Schwefel stehen, und $Z_3$ den Rest $R_1$ und $Z_4$ Wasserstoff bedeuten, oder $Z_1$ für den Rest $-Z-R_o$ steht, worin Z und $R_o$ die für die Reaktion $b_1$) gegebenen Bedeutungen haben, $Z_2$ und $Z_3$ zusammen eine Bindung bilden, und $Z_4$ den Rest $R_1$ bedeutet, ringschliesst, oder

c) ein Phosphinsäurehalogenid der Formel XII

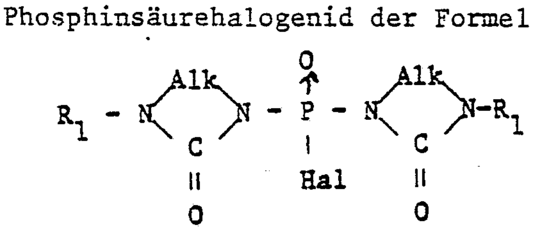

(XII) ,

worin Hal Halogen bedeutet, mit einer Amin-Verbindung der Formel $Py-NH-R_2$ (IVa) umsetzt, und, wenn erwünscht, eine erhaltene Verbindung der Formel I, in der der Rest Py ein Halogen enthält, enthalogeniert, und/oder, wenn erwünscht, eine erhaltene Verbindung in eine andere Verbindung der Erfindung umwandelt, und/oder, wenn erwünscht, eine erhaltene Base in ein Säureaddtionssalz oder ein erhaltenes Salz in die freie Base oder in ein anderes Säureadditionssalz überführt.

11. Verfahren nach Anspruch 10, dadurch gekennzeichnet, dass man in der Reaktion a) Ausgangsstoffe verwendet, in welchen eine abspaltbare Gruppe X oder Y eine im Anspruch 2 angegebene Gruppe ist.

12. Verfahren nach Anspruch 10, dadurch gekennzeichnet, dass man in der Reaktion $b_1$) oder $b_2$) Ausgangsstoffe verwendet, in welchen $R_o$ Niederalkyl bedeutet.

13. Verfahren nach einem der Ansprüche 10 bis 12, dadurch gekennzeichnet, dass man Verbindungen der allgemeinen Formel I, worin Py für gegebenenfalls durch einen, zwei oder drei gleiche oder verschiedene Substituenten der Gruppe Niederalkyl, Hydroxy, Niederalkoxy, Halogen, Amino, gegebenenfalls durch Niederalkyl, Niederalkoxy, Hydroxy, Amino, Niederalkylamino, Diniederalkylamino oder Halogen substituiertes Phenyl, Niederalkylamino, Diniederalkylamino, Pyrrolidino, Piperidino, Morpholino, Thiomorpholino, Niederalkanoylamino, Niederalkoxycarbonylamino, Ureido, 3-Niederalkylureido und 3,3,-Diniederalkylureido substituiertes, über ein Kohlenstoffatom mit dem Stickstoffatom verbundenes 4- oder 5-Pyrimidinyl steht, $R_1$ und $R_2$ unabhängig voneinander Wasserstoff, Niederalkyl oder Niederalkenyl bedeuten, und Alk für Niederalkylen steht, das die beiden Stickstoffatome durch 2 bis 4 Kohlenstoffatome trennt, wobei mit "nieder" bezeichnete Reste bis zu 4 Kohlenstoffatomen enthalten, ihre tautomeren Verbindungen und Säureadditionssalze herstellt.

14. Verfahren nach einem der Ansprüche 10 bis 12, dadurch gekennzeichnet, dass man Verbindungen der allgemeinen Formel I, worin Py für gegebenenfalls durch ein, zwei oder drei gleiche oder verschiedene Substituenten der Gruppe Niederalkyl, Niederalkoxy, Phenyl, Amino, Niederalkylamino, Diniederalkylamino oder Morpholino und/oder Halogen substituiertes, über ein Kohlenstoffatom mit dem Stickstoffatom verbundenes 4- oder 5-Pyrimidinyl steht, $R_1$ Wasserstoff oder Niederalkyl und $R_2$ Wasserstoff oder Niederalkyl darstellen, und Alk für Niederalkylen steht, das die beiden Stickstoffatome durch 2 bis 3 Kohlenstoffatome trennt, wobei mit "nieder" bezeichnete Reste bis zu 4 Kohlenstoffatomen enthalten, und Halogen ein Atomgewicht bis zu 35 aufweist, und ihre Säureadditionssalze herstellt.

15. Verfahren nach einem der Ansprüche 10 bis 12, dadurch gekennzeichmet, dass man Verbindungen der allgemeinen Formel II

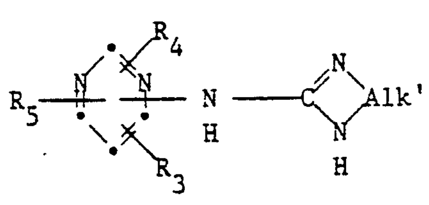

(II),

worin Alk' für Niederalkylen mit bis zu 4 Kohlenstoffatomen, das die beiden Stickstoffatome durch 2 bis 3 Kohlenstoffatome trennt, steht, und jeder der Reste $R_3$, $R_4$ und $R_5$ Wasserstoff, Niederalkyl mit bis zu 4 Kohlenstoffatomen oder Niederalkoxy mit bis zu 4 Kohlenstoffatomen, Halogen, Diniederalkylamino, Morpholino oder Phenyl, bedeutet, und ihre Säureadditionssalze herstellt.

16. Verfahren nach einem der Ansprüche 10 bis 12, dadurch gekennzeichnet, dass man Verbindungen der allgemeinen Formel III

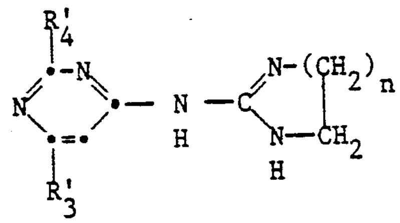

(III),

worin $R_3'$ und $R_4'$ unabhängig voneinander für Wasserstoff, Niederalkyl mit bis zu 4 Kohlenstoffatomen oder Niederalkoxy mit bis zu 4 Kohlenstoffatomen, Halogen oder Diniederalkylamino stehen, und n für 1 oder 2 steht, und ihre Säureadditionssalze herstellt.

17. Verfahren nach einem der Ansprüche 10 bis 12, dadurch gekennzeichnet, dass man 2-[(6-Chlor-2-dimethylamino-4-pyrimidinyl)-amino]-2-imidazolin und seine Säureadditionssalze herstellt.

18. Verfahren zur Herstellung eines pharmazeutischen Präparates, gekennzeichnet, durch die Verarbeitung eines erfindungsgemässen Wirkstoffs nach einem der Ansprüche 1 bis 9, mit einem pharamzeutischen Trägermaterial.

19. Verfahren zur Herstellung eines pharmazeutischen Präparates, gekennzeichnet durch die Verarbeitung eines erfindungsgemässen Wirkstoffs nach einem der Ansprüche 10 bis 17, mit einem pharmazeutischen Trägermaterial.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

0019811

Nummer der Anmeldung

EP 80 10 2724

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | FR - A - 2 068 499 (BEECHAM)<br>* Seiten 21 bis 24 *<br>---- | 1-3,5,6,10-15,18 |

KLASSIFIKATION DER ANMELDUNG (Int. Cl.[3])

C 07 D 403/12
239/42
A 61 K 31/505
C 07 D 239/48
239/46
239/52
239/54

RECHERCHIERTE SACHGEBIETE (Int. Cl.[3])

C 07 D 403/12
403/14
239/42

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-15,18

Unvollständig recherchierte Patentansprüche:

Nicht recherchierte Patentansprüche: 16,17

Grund für die Beschränkung der Recherche:

Verfahren zur chirurgischen oder therapeutischen Behandlung des menschlichen oder tierischen Körpers (Siehe Art. 52(4) des Europäischen Patentübereinkommens)

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| Den Haag | 04-09-1980 | FRANCOIS |

EPA Form 1505.1 06.78